(19) Europäisches Patentamt
European Patent Office
Office européen des brevets

(11) EP 4 000 603 A1

(12) EUROPEAN PATENT APPLICATION
published in accordance with Art. 153(4) EPC

(43) Date of publication:
**25.05.2022 Bulletin 2022/21**

(21) Application number: **21832972.0**

(22) Date of filing: **28.05.2021**

(51) International Patent Classification (IPC):
***A61K 9/127*** *(2006.01)* ***A61K 31/704*** *(2006.01)*
***A61K 36/185*** *(2006.01)* ***A61K 47/69*** *(2017.01)*
***A61P 37/00*** *(2006.01)*

(52) Cooperative Patent Classification (CPC):
**A61K 9/127; A61K 31/704; A61K 36/185; A61K 47/69; A61P 37/00**

(86) International application number:
**PCT/IB2021/054673**

(87) International publication number:
**WO 2022/003443 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 KR 20200080178**

(71) Applicant: **Eyegene, Inc.**
**Seoul 07528 (KR)**

(72) Inventors:
- **CHO, Yang Je**
  **Seoul 04423 (KR)**
- **LEE, Na Gyong**
  **Seoul 01913 (KR)**
- **KIM, Kwangsung**
  **Goyang-si Gyeonggi-do 10371 (KR)**
- **PARK, Shin Ae**
  **Ganghwa-gun Incheon 23048 (KR)**
- **AHN, Sunyoung**
  **Goyang-si Gyeonggi-do 10550 (KR)**

(74) Representative: **Mewburn Ellis LLP**
**Aurora Building**
**Counterslip**
**Bristol BS1 6BX (GB)**

# (54) COMPOSITION FOR INHIBITING SAPONIN-INDUCED HEMOLYSIS, CONTAINING CATIONIC LIPOSOME

(57) A cationic liposome having the effect of inhibition of red blood cell hemolysis induced by saponin is disclosed. More particularly, a composition for inhibiting red blood cell hemolysis by saponin comprising a cationic liposome containing an unsaturated lipid, a composition for immunity enhancement and a composition for drug delivery comprising the composition for inhibiting red blood cell hemolysis by saponin, and a drug delivery carrier and a drug-carrier complex comprising a cationic liposome containing an unsaturated lipid are disclosed.

Saponin exhibits a wide range of pharmacological and biological activities, such as anti-inflammatory activity, etc., including strong and effective immunological activity, and thus is effectively used medically and pharmaceutically, but has a disadvantage of causing hemolysis to red blood cells. Although saponin is generally used along with cholesterol, etc. to inhibit the hemolysis of saponin, it is confirmed herein that red blood cell hemolysis by saponin can be inhibited using a cationic liposome, which is more effective and economical in inhibiting the hemolysis of saponin. Therefore, saponin can be more usefully applied to the manufacture of immunity enhancers, drug delivery carriers, etc.

FIG. 14

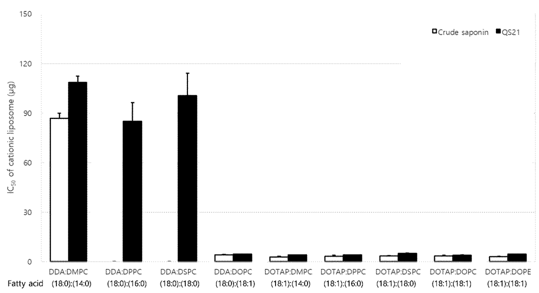


EP 4 000 603 A1

## Description

### TECHNICAL FIELD

The present invention relates to a cationic liposome having the effect of inhibition of red blood cell hemolysis induced by saponin, and more particularly to a composition for inhibiting red blood cell hemolysis by saponin comprising a cationic liposome containing an unsaturated lipid, a composition for immunity enhancement and a composition for drug delivery comprising the composition for inhibiting red blood cell hemolysis by saponin, and a drug delivery carrier and a drug-carrier complex comprising a cationic liposome containing an unsaturated lipid.

### BACKGROUND ART

Saponin is a glycoside compound that is produced as a secondary metabolite of steroid and triterpene. Saponin exhibits a wide range of pharmacological and biological activities, such as anti-inflammatory activity, etc., including strong and effective immunological activity. In general, saponin is known to have the effect of increasing the immune function, and saponin is used as an adjuvant for vaccines or as an anticancer agent (Newman MJ, et al., J. Immunol. 148:2357-2362, 1992; Sun HX, et al., Vaccine 27: 1787-1796, 2009). However, saponin induces a foaming action and a hemolytic action, "hemolytic action" meaning that red blood cells are destroyed and the contents (cytoplasm) thereof are dissolved into the surrounding liquid (e.g. plasma), which is called a hemolytic reaction or simply hemolysis. Hemolysis occurs because cholesterol in the red blood cell membrane binds strongly to saponin to thus destroy the membrane structure.

In order to use saponin for medicinal purposes, red blood cell hemolysis by saponin has to be inhibited, and cholesterol is usually used therefor. In general, cholesterol, which is an animal-derived ingredient, is subjected to strict management standards when manufacturing pharmaceuticals and the like, and in order to overcome this problem, semi-synthetic or synthetic cholesterol has recently been developed and used in the manufacture of pharmaceuticals, but is disadvantageous in that the price thereof is very high.

Technology for the safe and efficient delivery of various drugs has been studied for a long time. Encapsulation technology is used in order to maintain activity without deterioration during manufacture, distribution, etc. This technology is capable of minimizing drug deterioration and loss by encapsulating the drug in a carrier such as a liposome, and makes it possible to contain both hydrophilic and lipophilic materials therein. A capsule formulation serves as a drug delivery carrier and protector in pharmaceuticals, is used for gene therapy and cancer chemotherapy in the pharmaceutical field, and serves as an effective material delivery carrier and water delivery carrier in the cosmetic field. Encapsulation technology is also capable of playing a role in controlling the release rate as well as storing the contained material in a stable state.

A liposome is a self-assembled lipid-bilayer structure, and is an amphipathic molecule having both a hydrophobic portion and a hydrophilic portion. A liposome is excellent in biocompatibility, is simple to manufacture, and is advantageously capable of delivering water-soluble and fat-soluble drugs, so thorough research into liposomes as drug delivery carriers having fewer side effects in the body is ongoing (Kwang Jae Cho, Korean Journal of Otorhinolaryngology-Head and Neck Surgery 2007;50(7): 562-572). A liposome is chemically stable, non-irritating, nontoxic, and structurally similar to skin biolipid membranes. Moreover, since it may be manufactured by variously changing the surface properties, it may be used in various ways in cosmetics, pharmaceuticals, adjuvants, drug delivery systems, and the like.

Against this technical background, the present inventors have made great efforts to inhibit hemolysis that occurs when saponin is administered into the body while utilizing the therapeutic efficacy or immunity enhancement function of saponin, and thus have ascertained that, when saponin is mixed with a cationic liposome comprising an unsaturated cationic lipid or neutral lipid, a hemolytic phenomenon that occurs when saponin is administered alone may be effectively inhibited, thus culminating in the present invention.

The information described in the background section is only for improving understanding of the background of the present invention, and it is not to be construed as including information forming the related art already known to those of ordinary skill in the art to which the present invention belongs.

### SUMMARY OF THE INVENTION

It is an object of the present invention to provide a composition for inhibiting red blood cell hemolysis by saponin comprising a cationic liposome having the ability to inhibit red blood cell hemolysis by saponin.

It is another object of the present invention to provide a method of inhibiting red blood cell hemolysis by saponin comprising administering the composition to a subject, the use of the composition for inhibiting red blood cell hemolysis by saponin, and the use of the composition for the preparation of a therapeutic agent for inhibiting red blood cell hemolysis by saponin.

It is still another object of the present invention to provide a composition for immunity enhancement comprising the composition for inhibiting red blood cell hemolysis by saponin.

It is yet another object of the present invention to provide a method of enhancing immunity comprising administering the composition for immunity enhancement to a subject, the use of the composition for immunity enhancement for enhancing immunity, and the use of the composition for immunity enhancement for the preparation of a therapeutic agent for immunity enhancement.

It is a further object of the present invention to provide a composition for drug delivery comprising the composition for inhibiting red blood cell hemolysis by saponin.

It is still a further object of the present invention to provide a drug delivery carrier comprising a cationic liposome containing a cationic lipid and a neutral lipid, and a drug-carrier complex in which a drug is adsorbed to or encapsulated in a cationic liposome containing a cationic lipid and a neutral lipid.

In order to achieve the above objects, the present invention provides a composition for inhibiting red blood cell hemolysis by saponin comprising a cationic liposome, containing a cationic lipid and a neutral lipid, and saponin.

In addition, the present invention provides a method of inhibiting red blood cell hemolysis by saponin comprising administering the composition for inhibiting red blood cell hemolysis by saponin to a subject, the use of the composition for inhibiting red blood cell hemolysis by saponin to inhibit red blood cell hemolysis by saponin, and the use of the composition for inhibiting red blood cell hemolysis by saponin for the preparation of a therapeutic agent for inhibiting red blood cell hemolysis by saponin.

In addition, the present invention provides a composition for immunity enhancement comprising the composition for inhibiting red blood cell hemolysis by saponin.

In addition, the present invention provides a method of enhancing immunity comprising administering the composition for immunity enhancement to a subject, the use of the composition for immunity enhancement to enhance immunity, and the use of the composition for immunity enhancement for the preparation of a therapeutic agent for immunity enhancement.

In addition, the present invention provides a composition for drug delivery comprising the composition for inhibiting red blood cell hemolysis by saponin.

In addition, the present invention provides a drug delivery carrier comprising a cationic liposome containing a cationic lipid and a neutral lipid.

In addition, the present invention provides a drug-carrier complex in which a drug is adsorbed to or encapsulated in a cationic liposome containing a cationic lipid and a neutral lipid.

## BRIEF DESCRIPTION OF THE DRAWINGS

FIGS. 1 and 2 are graphs showing the ability of a liposome to inhibit hemolysis induced by crude saponin depending on the polarity thereof.

FIGS. 3 and 4 are graphs showing red blood cell hemolysis (%) depending on the concentration of *Quillaja* saponaria-derived crude saponin and QS21.

FIGS. 5 to 13 are graphs showing the effect of the liposome on inhibition of hemolysis of 2.5 μg of crude saponin and QS21.

FIG. 14 is a graph showing the effect of inhibition of hemolysis induced by saponin depending on the presence or absence of unsaturated fatty acid in the cationic lipid or neutral lipid in the cationic liposome.

FIG. 15 is a graph showing cytotoxicity depending on the presence or absence of unsaturated fatty acid in the cationic lipid or neutral lipid in the cationic liposome.

FIG. 16 is graphs showing red blood cell hemolysis (%) depending on the concentration of steroidal saponin.

FIGS. 17 to 20 are graphs showing the effect of the liposome on inhibition of hemolysis induced by steroidal saponin.

FIG. 21 is graphs showing red blood cell hemolysis (%) depending on the concentration of triterpenoid saponin.

FIGS. 22 to 25 are graphs showing the effect of the liposome on inhibition of hemolysis induced by triterpenoid saponin.

FIGS. 26 to 31 are results confirming the effect of the cationic liposome on inhibition of hemolysis induced by saponin depending on the ratio of cationic and neutral lipids in the cationic liposome.

## DETAILED DESCRIPTION OF THE INVENTION

Unless otherwise defined, all technical and scientific terms used herein have the same meanings as those typically understood by those skilled in the art to which the present invention belongs. Generally, the nomenclature used herein and the test method described below are well known in the art and are typical.

Liposomes are variously classified depending on properties such as surface charge, size, membrane structure, and the like. For example, the surface charge of liposomes is determined by combinations of various lipid materials, such as neutral lipids, cationic lipids, anionic lipids, etc.

In an embodiment of the present invention, a cationic liposome capable of inhibiting red blood cell hemolysis by saponin is developed, and it is confirmed to be effectively applicable to the manufacture of a drug delivery carrier as well as a formulation for immunity enhancement using saponin, even without the use of cholesterol, which is generally used to suppress the hemolysis induced by saponin. This promises the safe use of saponin for medical and pharmaceutical purposes.

Accordingly, in one aspect, the present invention is directed to a composition for inhibiting red blood cell hemolysis by saponin comprising a cationic liposome and saponin, in which the cationic liposome contains a cationic lipid and a neutral lipid.

As used herein, the term "lipid" includes a fatty acid, phospholipid, fatty acid ester, steroid, and the like. The term "unsaturated lipid" refers to a lipid including at least one carbon-carbon double bond in the fatty acid chain included in the lipid.

In the present invention, the cationic lipid or neutral lipid may comprise at least one unsaturated fatty acid.

In the present invention, the cationic lipid or neutral lipid may comprise at least one unsaturated fatty acid chain, and each unsaturated fatty acid chain has 10 to 20 carbon atoms, preferably 14 to 18 carbon atoms, and the number of carbon-carbon double bonds included in each fatty acid chain is 1 to 6, preferably 1.

Any one of the cationic lipid and the neutral lipid included in the composition for inhibiting hemolysis according to the present invention may be an unsaturated lipid, and the remaining one thereof may be an unsaturated lipid or a saturated lipid.

In the present invention, the cationic lipid may be selected from the group consisting of 1,2-dioleoyl-3-(trimethylammonium)propane (DOTAP), dimethyldioctadecylammonium bromide (DDA), 3β-[N-(N',N'-dimethylaminoethane)carbamoyl]cholesterol (DC-Chol), 1,2-dioleoyl-3-(dimethylammonium)propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0-18:1 Ethyl PC), 1,2-dioleoyl-sn-glycero-3-ethylphosphocholine (18:1 Ethyl PC), 1,2-distearoyl-sn-glycero-3-ethylphosphocholin (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholin (12:0 Ethyl PC), N1-[2-((1S)-1-[(3-aminopropyl)amino]-4-[di(3-amino-propyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TA), 1,2-dimyristoyl-3-trimethylammonium-propane (14:0 TAP), and N4-cholesteryl-spermine (GL67).

The cationic lipid may include a lipid in which a cationic functional group is introduced into a cholesterol derivative, etc.

In the present invention, the neutral lipid may be selected from the group consisting of 1,2-dimyristoyl-sn-glycero-3-phosphatidylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphatidylethanolamine (PE), phosphatidylglycerol (PG), phosphoric acid (PA), and phosphatidylcholine (PC), but is not limited thereto.

Preferably, the cationic lipid is DOTAP or DDA, and the neutral lipid is DMPC, DOPC, DOPE, DPPC, or DSPC, but the present invention is not limited thereto.

In the present invention, the weight ratio (%) of the cationic lipid in the cationic liposome may be 10 to 100%, but is not limited thereto. In the present invention, "weight ratio" is used in the same meaning as "amount".

In an embodiment of the present invention, it can be confirmed that the amount of the cationic lipid DDA is 30% to 70% and that the amount of the cationic lipid DOTAP is 10% to 100% in the cationic liposomes that inhibit hemolysis of saponin.

In the present invention, the liposome may further comprise a glycolipid, and the glycolipid may be at least one selected from the group consisting of digalactosyldiglyceride, galactosyldiglyceride sulfuric acid ester, and sphingoglycolipids such as galactosylceramide, galactosylceramide sulfuric acid ester, lactosylceramide, ganglioside G7, ganglioside G6, and ganglioside G4, but is not limited thereto.

The liposome may further comprise a sterol derivative, and the sterol derivative may be at least one selected from the group consisting of cholesterol, dihydrocholesterol, cholesterol ester, phytosterol, sitosterol, stigmasterol, campesterol, cholestanol, lanosterol, 1-O-sterolglucoside, 1-O-sterolmaltoside, and 1-O-sterolgalactoside, but is not limited thereto.

The liposome may further comprise a glycol derivative, and the glycol derivative may be at least one selected

from the group consisting of ethylene glycol, diethylene glycol, triethylene glycol, propylene glycol, dipropylene glycol, trimethylene glycol, and 1,4-butanediol, but is not necessarily limited thereto.

The liposome may further comprise an aliphatic amine, and the aliphatic amine may be at least one selected from the group consisting of stearylamine, octylamine, oleylamine, and linoleylamine, but is not necessarily limited thereto.

Methods of manufacturing liposomes may be classified into 'top-down methods' including forming large-sized liposomes and then dividing the same into small-sized liposomes, and 'bottom-up methods' including assembling small-size liposomes using lipid monomers. In order to manufacture a liposome using the top-down method, dissolving a lipid in an organic solvent, removing the organic solvent, and rehydrating the lipid with an aqueous solution are performed.

A typical method of manufacturing a liposome may include a film-rehydration method or a lipid hydration method. Using such a method, LUV is formed and is then physically disrupted using a homogenizer, a microfluidizer, or a high-pressure homogenizer, thereby manufacturing a liposome having a desired size.

The cationic liposome of the present invention may be manufactured using a thin film method, an injection method, or a freeze-drying method, but the present invention is not limited thereto.

The thin film method is performed in a manner in which a lipid is dissolved in an organic solvent and dried to form a membrane, which is then added with a solution to afford a cationic liposome, the injection method is performed in a manner in which an organic solvent containing a lipid is dropped using a syringe to afford a cationic liposome, and the freeze-drying method is performed in a manner in which a lipid is dissolved in an organic solvent and is freeze-dried to thus volatilize the organic solvent, followed by rehydrating the lipid with a solution to afford a cationic liposome.

In the present invention, the liposome thus manufactured may be optionally freeze-dried for ease of storage. Cake, plaque, or powder formed by freeze-drying the liposome may be administered after reconstitution with sterile water when used.

The liposome manufactured using the method of the present invention may be used for injection, transdermal delivery, transnasal delivery, and pulmonary delivery of a drug. The technology required for such formulation and pharmaceutically appropriate carriers, additives and the like, are widely known to those of ordinary skill in the art of pharmaceuticals. In this regard, reference may be made to Remington's Pharmaceutical Sciences (19th ed., 1995).

In the present invention, saponin may be adsorbed to the cationic liposome through electrostatic attraction, but the present invention is not limited thereto. Alternatively, saponin may be contained within the cationic liposome, or saponin may be bound to the lipid membrane of the cationic liposome.

As used herein, the term "adsorbed" means that a material is bound to the inside or outside of the liposome, and the form of binding is not particularly limited, so long as saponin according to the present invention is able to be effectively delivered.

In the present invention, saponin may be selected from the group consisting of ginsenoside Rb1, digitonin, β-aescin, *Quillaja* saponaria-derived crude saponin and fractions thereof, QS21, Quil A, QS7, QS18, QS17 and salts thereof, steroidal saponin, and triterpenoid saponin, but is not limited thereto. The steroidal saponin may include digitonin or Paris VII, and the triterpenoid saponin may include aescin, α-hederin, hederagenin, echinocystic acid, chrysanthellin A, chrysanthellin B, bayogenin, medicagenic acid, maslinic acid, oleanolic acid, erythrodiol, or asiatic acid. Preferably, the saponin is *Quillaja* saponaria-derived crude saponin, QS21, digitonin, Paris VII, aescin, or α-hederin.

The composition for inhibiting red blood cell hemolysis by saponin according to the present invention may comprise a pharmaceutically effective amount of the cationic liposome alone, or may further comprise at least one pharmaceutically acceptable carrier, excipient, or diluent. The "pharmaceutically effective amount" is an amount sufficient to inhibit red blood cell hemolysis by saponin.

The term "pharmaceutically acceptable" means that the compound is physiologically acceptable and does not usually cause gastrointestinal disorders, allergic reactions such as dizziness, or similar reactions when administered to humans. Examples of the carrier, excipient and diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. Moreover, fillers, anti-agglomeration agents, lubricants, wetting agents, fragrances, emulsifiers, and preservatives may be additionally included.

The composition for inhibiting red blood cell hemolysis by saponin according to the present invention may be formulated using a method known in the art so as to provide rapid, sustained or delayed release of the active ingredient after administration to mammals other than humans. Formulations may be in the form of powders, granules, tablets, emulsions, syrups, aerosols, soft or hard gelatin capsules, sterile injectable solutions, freeze-dried powders, and sterile powders.

The composition for inhibiting red blood cell hemolysis by saponin according to the present invention may be administered through various routes, including oral, transdermal, subcutaneous, intravenous or intramuscular administration, and the dosage of the active ingredient may be appropriately selected depending on various factors such as the route of administration, the patient's age, gender, and weight, severity of disease, and the like.

In another aspect, the present invention is directed to a method of inhibiting red blood cell hemolysis by saponin

comprising administering the composition for inhibiting red blood cell hemolysis by saponin to a subject.

In another aspect, the present invention is directed to the use of the composition for inhibiting red blood cell hemolysis by saponin to inhibit red blood cell hemolysis by saponin.

In another aspect, the present invention is directed to the use of the composition for inhibiting red blood cell hemolysis by saponin for the preparation of a therapeutic agent for inhibiting red blood cell hemolysis by saponin.

In the present invention, the method and the use comprise the composition for inhibiting red blood cell hemolysis by saponin described above, and thus a description that overlaps the above description of the composition for inhibiting hemolysis according to the present invention will be omitted.

In another aspect, the present invention is directed to a composition for immunity enhancement comprising the composition for inhibiting red blood cell hemolysis by saponin.

In another aspect, the present invention is directed to a method of enhancing immunity comprising administering the composition for immunity enhancement to a subject.

In another aspect, the present invention is directed to the use of the composition for immunity enhancement to enhance immunity.

In another aspect, the present invention is directed to the use of the composition for immunity enhancement for the preparation of a therapeutic agent for immunity enhancement.

In the present invention, the composition for immunity enhancement, the method of enhancing immunity, and the use of the composition for immunity enhancement comprise the composition for inhibiting red blood cell hemolysis by saponin described above, and thus a description that overlaps the above description of the composition for inhibiting red blood cell hemolysis by saponin according to the present invention will be omitted.

As used herein, the term "immunity enhancement" means inducing an initial immune response or measurably increasing an existing immune response to an antigen.

In the present invention, the composition for enhancing immunity may be used alone or in combination with an adjuvant to form a pharmaceutical composition.

The adjuvant may include, for example, a Group 2 element selected from the group consisting of Mg, Ca, Sr, Ba and Ra or a salt thereof; a Group 4 element selected from the group consisting of Ti, Zr, Hf and Rf; a salt of aluminum or a hydrate thereof; or dimethyloctadecylammonium bromide. The salt may be formed with, for example, oxide, peroxide, hydroxide, carbonate, phosphate, pyrophosphate, hydrogen phosphate, dihydrogen phosphate, sulfate, and silicate.

Also, the adjuvant may include, for example, a PRR (pattern recognition receptor) agonist selected from the group consisting of a TLR (Toll-like receptor) agonist, an RLR (RIG-I-like receptor) agonist, and an NLR (NOD-like receptor) agonist.

In another aspect, the present invention is directed to a composition for drug delivery comprising the composition for inhibiting red blood cell hemolysis by saponin.

In another aspect, the present invention is directed to a drug delivery carrier comprising a cationic liposome containing a cationic lipid and a neutral lipid.

In another aspect, the present invention is directed to a drug-carrier complex in which a drug is adsorbed to or encapsulated in a cationic liposome containing a cationic lipid and a neutral lipid.

In the present invention, the cationic lipid or neutral lipid may comprise at least one unsaturated fatty acid.

In the present invention, the composition for drug delivery, the drug delivery carrier, and the drug-carrier complex comprises the cationic liposome containing the cationic lipid and the neutral lipid as described above, and thus a description that overlaps the above description of the cationic liposome containing the cationic lipid and the neutral lipid according to the present invention will be omitted.

In the present invention, the drug may be applied without limitation, so long as it is able to be delivered using the cationic liposome according to the present invention, such as a protein, gene, peptide, compound, antigen, or natural material.

The composition for immunity enhancement or the composition for drug delivery according to the present invention may further comprise an appropriate excipient and diluent typically used in the manufacture of pharmaceutical compositions (Remington's Pharmaceutical Science, Mack Publishing Co., Easton PA). Moreover, the composition may be formulated in oral dosage forms such as powders, granules, tablets, capsules, suspensions, emulsions, syrups, aerosols, and the like and in the form of sterile injectable solutions according to individual typical methods.

Examples of the carrier, excipient and diluent that may be included in the composition for immunity enhancement or the composition for drug delivery may include lactose, dextrose, sucrose, sorbitol, mannitol, xylitol, maltitol, starch, glycerin, acacia gum, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

The composition may be formulated using typically used diluents or excipients such as fillers, extenders, binders, wetting agents, disintegrants, surfactants, etc. Solid formulations for oral administration may include tablets, pills, powders, granules, capsules, etc., and such solid formulations may be manufactured using at least one excipient, for example,

starch, calcium carbonate, sucrose, lactose, gelatin, etc. In addition to simple excipients, lubricants such as magnesium stearate and talc may also be used. As liquid formulations for oral administration, suspensions, internal solutions, emulsions, syrups, etc. may be used. In addition to water and liquid paraffin, which are commonly used simple diluents, various excipients such as wetting agents, sweeteners, fragrances, preservatives, etc. may be included. Formulations for parenteral administration may include sterile aqueous solutions, non-aqueous formulations, suspensions, emulsions, and freeze-dried formulations.

For the non-aqueous formulations and suspensions, propylene glycol, polyethylene glycol, vegetable oils such as olive oil, injectable esters such as ethyl oleate, and the like may be used.

The dosage of the composition for immunity enhancement or the composition for drug delivery according to the present invention may vary depending on the age, gender, weight, etc. of a subject, and the dosage may be increased or decreased depending on the route of administration, the severity of disease, gender, weight, age, etc.

Hereinafter, the present invention will be described in more detail with reference to examples. However, it will be obvious to those skilled in the art that these examples are provided only for illustration of the present invention and should not be construed as limiting the scope of the present invention.

## Example 1: Materials

The lipids, which are materials for the liposomes used in the following Examples, are shown in Table 1 below.

[Table 1]

| | Type of Lipid | Fatty acid | Charge (pH 7.4) | Structure |
|---|---|---|---|---|
| Cationic lipid | **DDA** (Dimethyldioctadecylammonium) | 18:0 | + 1 | 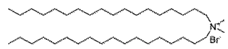 |
| | **DOTAP** (1,2-dioleoyl-3-trimethylammonium-propane) | 18:1 | + 1 | 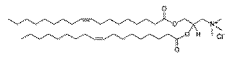 |
| Neutral lipid | **DMPC** (1,2-dimyristoyl-sn-glycero-3-phosphocholine) | 14:0 | 0 | 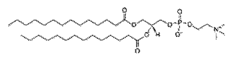 |
| | **DPPC** (1,2-dipalmitoyl-sn-glycero-3-phosphocholine) | 16:0 | 0 | 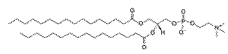 |
| | **DSPC** (1,2-distearoyl-sn-glycero-3-phosphocholine) | 18:0 | 0 | 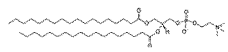 |
| | **DOPC** (1,2-dioleoyl-sn-glycero-3-phosphocholine) | 18:1 | 0 | 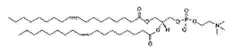 |
| | **DOPE** (1,2-dioleoyl-sn-glycero-3-phosphoethanolamine) | 18:1 | 0 | 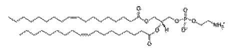 |
| Anionic lipid | **DMPG** (1,2-dimyristoyl-sn-glycero-3-phospho-(1'-rac-glycerol)) | 14:0 | -1 | 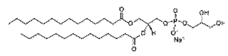 |

In Table 1, (18:0), (18:1), (14:0), (16:0), etc. represent the degrees of saturation of respective lipids, "N:0" represents a completely saturated lipid, and "N:1" represents a lipid that is unsaturated at a ratio of 1 relative to N carbons.

The sources of lipids were as follows:

Cationic lipid: DOTAP (Merck), DDA (Sigma-Aldrich)

Neutral lipid: DMPC (Corden Pharma), DOPC, DOPE, DSPC, DPPC (Avanti Polar Lipids)

Anionic lipid: DMPG (Avanti Polar Lipids)

## Example 2: Comparison of ability of liposome to inhibit hemolysis of crude saponin depending on polarity thereof

In order to confirm the ability of the liposome to inhibit hemolysis of crude saponin depending on the polarity thereof, cationic, neutral and anionic liposomes were manufactured using a freeze-drying method, and hemolysis analysis

was performed.

Example 2-1: Manufacture of liposomes

The types of liposomes used in this Example are shown in Table 2 below.

[Table 2]

| No. | Liposome | Weight ratio (%) | Conc. (mg/mL) |
|---|---|---|---|
| 1 | DDA:DOPC | 50:50 | 2 |
| 2 | DOPC | 100 | 2 |
| 3 | DMPG:DOPC | 50:50 | 2 |

The liposome shown in Table 2 was manufactured using the following method.
1) 40 mg of each of DDA, DOPC and DMPG was weighed and placed in a 70 mL glass vial.
2) 20 mL of t-butyl alcohol was placed in each vial to prepare a 2 mg/mL lipid stock solution, which was then heated in a water bath at 65°C for 10 minutes, thus completely dissolving the lipid.
3) Each lipid mixture was prepared by mixing DDA, DOPC, and DMPG in a 10 mL glass vial, as shown in Table 3 below.

[Table 3]

| DDA:DOPC | | DOPC | DMPG:DOPC | |
|---|---|---|---|---|
| DDA | DOPC | DOPC | DMPG | DOPC |
| 5 mL | 5 mL | 10 mL | 5 mL | 5 mL |

4) The inlet of each vial was covered with sealing tape, after which vortex mixing was performed for 5 seconds, and dense holes were formed in the sealing tape using a syringe needle.
5) The mixture was frozen in a freezer at -70°C for 2 hours and then transferred to a freeze dryer, followed by freeze-drying at 20 Pa and -80°C for about 18 hours to thus volatilize the organic solvent.
6) The resulting liposome cake was kept refrigerated until use and hydrated with 10 mL of sucrose in a HEPES buffer (pH 7.4) per vial for use in the experiment.

Example 2-2: Method of hemolysis analysis

1) The liposome, saponin, sucrose in a HEPES buffer (pH 7.4), and distilled water (D.W.) were placed in a 96-well plate, as shown in Tables 4 and 5 below.

[Table 4]

| **With saponin** (liposome concentration: 2 mg/mL, saponin concentration: 1 mg/mL) | | | | | | | | | | |
|---|---|---|---|---|---|---|---|---|---|---|
| **Group** | **Liposome conc. (pg/well)** | | | | | | | | **100% Hemolysis control** | **0% Hemolysis control** |
| | **0** | **10** | **20** | **40** | **50** | **60** | **80** | **100** | | |
| Liposome (μl) | 0 | 5 | 10 | 20 | 25 | 30 | 40 | 50 | 0 | 0 |
| Saponin (μl) | 10 | 10 | 10 | 10 | 10 | 10 | 10 | 10 | 0 | 0 |
| Buffer (μl) | 90 | 85 | 80 | 70 | 65 | 60 | 50 | 40 | 0 | 100 |
| D.W. (μl) | 0 | 0 | 0 | 0 | 0 | 0 | 0 | 0 | 100 | 0 |

[Table 5]

| Without saponin | | | | | | | | | | |
|---|---|---|---|---|---|---|---|---|---|---|
| Group | Liposome conc. (μg/well) | | | | | | | | 100% Hemolysis control | 0% Hemolysis control |
| | 0 | 10 | 20 | 40 | 50 | 60 | 80 | 100 | | |
| Liposome (μl) | 0 | 5 | 10 | 20 | 25 | 30 | 40 | 50 | 0 | 0 |
| Saponin (μl) | 0 | 0 | 0 | 0 | 0 | 0 | 0 | 0 | 0 | 0 |
| Buffer (μl) | 90 | 85 | 80 | 70 | 65 | 60 | 50 | 40 | 0 | 100 |
| D.W. (μl) | 10 | 10 | 10 | 10 | 10 | 10 | 10 | 10 | 100 | 0 |

2) The reaction was carried out at room temperature and 300 rpm for 30 minutes using an orbital shaker.
3) 3 mL of red blood cells (RBCs) were suspended in 10 mL of PBS and centrifuged at room temperature and 1500 rpm for 5 minutes, after which the supernatant was removed.
4) Steps 2) and 3) were repeated three times, and thus the RBCs were washed.
5) The RBC pellet was suspended in 1 mL of PBS and diluted to 1/100, followed by cell counting.
6) The RBCs were diluted to $1.5\times10^9$ cells/mL using PBS and dispensed at $3\times10^7$ cells/20 μL/well in a 96-well plate treated with the test material of 2) above.
7) The reaction was carried out at room temperature and 300 rpm for 1 hour using an orbital shaker.
8) The 96-well plate was centrifuged at room temperature and 1500 rpm for 5 minutes.
9) 50 μL of the supernatant was transferred to a new 96-well plate, and the absorbance was then measured at 415 nm.
10) Hemolysis (%) was calculated using the following equation.

$$\text{Hemolysis (\%)} = (\text{OD of sample} - \text{OD of 0\% hemolysis})/(\text{OD of 100\% hemolysis} - \text{OD of 0\% hemolysis}) \times 100$$

Example 2-3: Results of hemolysis analysis

The effect of the polarity of the liposome on inhibition of hemolysis induced by 10 μg of crude saponin was confirmed.

As a result, the neutral liposome (DOPC) and the anionic liposome (DMPG:DOPC) did not inhibit hemolysis induced by crude saponin (FIG. 1), whereas 100 μg of the cationic liposome (DDA:DOPC) inhibited 85% of hemolysis induced by crude saponin (FIG. 2).

**Example 3: Screening of cationic liposomes for inhibition of hemolysis induced by saponin**

In order to confirm the effects of the cationic liposomes on inhibition of hemolysis of saponin depending on the degree of unsaturation of cationic and neutral lipids, various liposomes were manufactured using a lipid film method, and hemolysis and cytotoxicity (HaCaT, L6, J774A.1) thereof were analyzed.

Example 3-1: Manufacture of liposomes

The types of liposomes used in this Example are shown in Table 6 below.

[Table 6]

| No. | Liposome | Weight ratio (%) | Conc. (mg/mL) |
|---|---|---|---|
| 1 | DDA:DMPC | 50:50 | 2 |
| 2 | DDA:DPPC | | |
| 3 | DDA:DSPC | | |
| 4 | DDA:DOPC | | |

(continued)

| No. | Liposome | Weight ratio (%) | Conc. (mg/mL) |
|---|---|---|---|
| 5 | DOTAP:DMPC | 50:50 | 2 |
| 6 | DOTAP:DPPC | | |
| 7 | DOTAP:DSPC | | |
| 8 | DOTAP:DOPC | | |
| 9 | DOTAP:DOPE | | |

The liposomes shown in Table 6 were manufactured using the following method.
1) Each of a cationic lipid and a neutral lipid was weighed and placed in a glass tube.
2) Chloroform (Daejung) was added thereto such that the lipid concentration was 2 mg/mL, and was completely dissolved at 37°C for 10 minutes to afford a lipid solution.
3) Each lipid mixture was prepared by mixing a cationic lipid solution and a neutral lipid solution at a weight ratio of 1:1 in a round-bottom flask.
4) Using a rotary evaporator, volatilization was performed for 30 minutes at 60°C for the lipid mixture containing DOTAP and at 80°C for the lipid mixture containing DDA, and thus whether chloroform did not remain and a lipid membrane film was formed on the wall of the flask was observed.
5) Sucrose in a HEPES buffer (pH 7.4) was placed in the flask such that the liposome concentration was 2 mg/mL, and the lipid membrane was dissolved at 65°C.
6) The liposome thus manufactured was dispensed in an amount of 500 μL into each glass vial, the inlet of the vial was closed with a rubber stopper, and the vial was placed in a freeze dryer (IlShinBioBase/Lyoph-pride10, SXX2), followed by freeze-drying, as shown in Table 7 below.

[Table 7]

| Step | State | Temperature (°C) | Time (hr) | Degree of vacuum (mTorr) |
|---|---|---|---|---|
| 1 | Pre-freezing | -40 | 2 | 999 |
| 2 | Maintaining temperature | -40 | 27 | 50 |
| 3 | Elevating temperature | -20 | 10 | 50 |
| 4 | Maintaining temperature | -20 | 2 | 50 |
| 5 | Elevating temperature | 20 | 10 | 50 |
| 6 | Maintaining temperature | 20 | 13 | 50 |

7) The liposome thus manufactured was stored in a refrigerator at 4°C until the test.

Example 3-2: Method of hemolysis analysis

1) After freeze-drying, the liposome stored in the glass vial was hydrated with 225 μL of distilled water and then allowed to react at 60°C for 10 minutes to thus completely dissolve the liposome.
2) The liposome, saponin, sucrose in a HEPES buffer (pH 7.4), and distilled water were placed in a 96-well plate, as shown in Tables 8 and 9 below.

[Table 8]

| **With saponin** (liposome concentration: 4.4 mg/mL, saponin concentration: 0.25 mg/mL) | | | | | | | | | | |
|---|---|---|---|---|---|---|---|---|---|---|
| **Group** | **Liposome conc. (pg/well)** | | | | | | | | **100% Hemolysi s control** | **0% Hemolysi s control** |
| | **0** | **2** | **3** | **6** | **13** | **25** | **50** | **100** | | |
| Liposom e (μl) | 0.0 | 0.4 | 0.7 | 1.4 | 2.8 | 5.6 | 11. 3 | 22. 5 | 0 | 0 |

(continued)

| With saponin (liposome concentration: 4.4 mg/mL, saponin concentration: 0.25 mg/mL) | | | | | | | | | | |
|---|---|---|---|---|---|---|---|---|---|---|
| Group | Liposome conc. (pg/well) | | | | | | | | 100% Hemolysi s control | 0% Hemolysi s control |
| | 0 | 2 | 3 | 6 | 13 | 25 | 50 | 100 | | |
| Saponin (μl) | 10.0 | 10. 0 | 10. 0 | 10. 0 | 10. 0 | 10. 0 | 10. 0 | 10. 0 | 0 | 0 |
| Buffer (μl) | 100. 0 | 99. 6 | 99. 3 | 98. 6 | 97. 2 | 94. 4 | 88. 8 | 77. 5 | 0 | 110 |
| D.W. (μl) | 0.0 | 0.0 | 0.0 | 0.0 | 0.0 | 0.0 | 0.0 | 0.0 | 110 | 0 |

[Table 9]

| Without saponin | | | | | | | | | | |
|---|---|---|---|---|---|---|---|---|---|---|
| Group | Liposome conc. (pg/well) | | | | | | | | 100% Hemolysi s control | 0% Hemolysi s control |
| | 0 | 2 | 3 | 6 | 13 | 25 | 50 | 100 | | |
| Liposom e (μl) | 0.0 | 0.4 | 0.7 | 1.4 | 2.8 | 5. 6 | 11. 3 | 22. 5 | 0 | 0 |
| Saponin (μl) | 0.0 | 0.0 | 0.0 | 0.0 | 0.0 | 0.0 | 0.0 | 0.0 | 0 | 0 |
| Buffer (μl) | 100. 0 | 99. 6 | 99. 3 | 98. 6 | 97. 2 | 94. 4 | 88. 8 | 77. 5 | 0 | 110 |
| D.W. (μl) | 10.0 | 10. 0 | 10. 0 | 10. 0 | 10. 0 | 10. 0 | 10. 0 | 10. 0 | 110 | 0 |

3) The reaction was carried out at room temperature and 300 rpm for 30 minutes using an orbital shaker.
4) 3 mL of RBCs were suspended in 10 mL of PBS and centrifuged at room temperature and 1500 rpm for 5 minutes, after which the supernatant was removed.
5) Steps 3) and 4) were repeated three times, and thus the RBCs were washed.
6) The RBC pellet was suspended in 1 mL of PBS and diluted to 1/100, followed by cell counting.
7) The RBCs were diluted to $1.5\times10^9$ cells/mL using PBS and dispensed at $3\times10^7$ cells/20 μL/well in a 96-well plate treated with the test material of 2) above.
8) The reaction was carried out at room temperature and 300 rpm for 1 hour using an orbital shaker.
9) The 96-well plate was centrifuged at room temperature and 1500 rpm for 5 minutes.
10) 50 μL of the supernatant was transferred to a new 96-well plate, and the absorbance was then measured at 415 nm.
11) Hemolysis (%) was calculated using the following equation.

$$\text{Hemolysis (\%)} = (\text{OD of sample} - \text{OD of 0\% hemolysis})/(\text{OD of 100\% hemolysis} - \text{OD of 0\% hemolysis}) \times 100$$

Example 3-3: Confirmation of hemolytic concentration for saponin

Based on the results of confirmation of hemolysis depending on the concentration of *Quillaja saponaria*-derived crude saponin (VET-SAP®, Desert King) and QS21 (Desert King), 100% hemolysis was observed when the amount of crude saponin was 2.5 μg or when the amount of QS21 was 2.5 μg (FIGS. 3 and 4) .

Example 3-4: Confirmation of effect of cationic liposome on inhibition of hemolysis induced by saponin

The type and concentration of the cationic liposome that inhibits hemolysis induced by 2.5 μg of crude saponin or QS21 were observed.

As a result, DDA:DMPC, DDA:DPPC, and DDA:DSPC were found not to inhibit hemolysis induced by crude

saponin or QS21 up to 100% (FIGS. 5 to 7). On the other hand, hemolysis induced by crude saponin or QS21 was 100% inhibited when using 25 μg of DDA:DOPC (liposome:saponin = 10:1), 50 μg of DOTAP:DMPC (liposome:saponin = 20:1), 50 μg of DOTAP:DPPC (liposome:saponin = 20:1), 50 μg of DOTAP:DSPC (liposome:saponin = 20:1), 25 μg of DOTAP:DOPC (liposome:saponin = 10:1), or 12.5 μg of DOTAP:DOPE (liposome:saponin = 5:1) (FIGS. 8 to 13).

In addition, the amount of cationic liposome that inhibits hemolysis induced by 2.5 μg of crude saponin or QS21 was confirmed using $IC_{50}$ (liposome concentration that inhibited 50% of hemolysis) and $IC_{100}$ (liposome concentration that inhibited 100% of hemolysis) (Tables 10 and 11).

[Table 10]

| Cationic liposome | | | Amount of cationic liposome that inhibits hemolysis induced by 2.5 μg of crude saponin (μg) | |
|---|---|---|---|---|
| Type | Lipid | | $IC_{50}$ | $IC_{100}$ |
| | Cationic | Neutral | | |
| DDA:DMPC | 18:0 | 14:0 | 199.9 | - |
| DDA:DPPC | 18:0 | 16:0 | - | - |
| DDA:DSPC | 18:0 | 18:0 | - | - |
| DDA:DOPC | 18:0 | 18:1 | 4.4 | 25.0 |
| DOTAP: DMPC | 18:1 | 14:0 | 2.8 | 50.0 |
| DOTAP: DPPC | 18:1 | 16:0 | 2.7 | 50.0 |
| DOTAP: DSPC | 18:1 | 18:0 | 3.3 | 50.0 |
| DOTAP: DOPC | 18:1 | 18:1 | 3.3 | 25.0 |
| DOTAP: DOPE | 18:1 | 18:1 | 2.9 | 12.5 |

[Table 11]

| Cationic liposome | | | Amount of cationic liposome that inhibits hemolysis induced by 2.5 μg of QS21 (μg) | |
|---|---|---|---|---|
| Type | Lipid | | $IC_{50}$ | $IC_{100}$ |
| | Cationic | Neutral | | |
| DDA:DMPC | 18:0 | 14:0 | 74.4 | - |
| DDA:DPPC | 18:0 | 16:0 | 75.5 | - |
| DDA:DSPC | 18:0 | 18:0 | 53.9 | - |
| DDA:DOPC | 18:0 | 18:1 | 4.4 | 25.0 |
| DOTAP: DMPC | 18:1 | 14:0 | 3.8 | 50.0 |
| DOTAP: DPPC | 18:1 | 16:0 | 3.9 | 50.0 |
| DOTAP: DSPC | 18:1 | 18:0 | 4.8 | 50.0 |
| DOTAP: DOPC | 18:1 | 18:1 | 3.8 | 25.0 |

(continued)

| Cationic liposome | | | Amount of cationic liposome that inhibits hemolysis induced by 2.5 μg of QS21 (μg) | |
|---|---|---|---|---|
| Type | Lipid | | $IC_{50}$ | $IC_{100}$ |
| | Cationic | Neutral | | |
| DOTAP: DOPE | 18:1 | 18:1 | 4.3 | 12.5 |

Consequently, unlike the neutral liposome containing only the neutral lipid, the cationic liposome containing the cationic lipid was confirmed to have hemolysis inhibitory activity, and the hemolysis inhibitory activity of liposomes was remarkably varied depending on the combination of the degrees of saturation of lipids contained in the cationic liposomes. Specifically, it can be confirmed that the higher the amount of the unsaturated fatty acid in the cationic liposome, the better the effect of inhibition of hemolysis induced by saponin (FIG. 14).

**Example 4: Analysis of cytotoxicity of liposome**

Example 4-1: Analysis method

1) HaCaT cells (human keratinocytes) (high-glucose DMEM, 10% FBS, 1% penicillin-streptomycin) were cultured in an incubator at 37°C and 5% $CO_2$.
2) When the cells reached 80-90% confluency in a 100 mm dish, the cell culture was removed through suction, and the cells were washed with 5 mL of PBS.
3) 5 mL of a trypsin-EDTA solution was placed in the 100 mm dish and allowed to react at 37°C and 5% $CO_2$ for 3 to 6 minutes, after which 5 mL of a cell culture medium was added thereto, and the cell suspension was transferred to a 15 mL tube and then centrifuged at 1,500 rpm and room temperature for 3 minutes, followed by removing the supernatant.
4) The cells were suspended in 10 mL of PBS and centrifuged at 1,500 rpm and room temperature for 3 minutes, after which the supernatant was removed.
5) The above steps were repeated two times, and thus the cells were washed.
6) The cells thus obtained were added with 3 mL of a cell culture medium to suspend the cells, followed by cell counting to determine the cell number and viability through staining with trypan blue.
7) The cell viability was confirmed to be 85% or more, after which the cell suspension was adjusted to a concentration of $1\times10^5$ cells/mL using the culture medium, dispensed at 100 μL/well in a 96-well plate, and cultured at 37°C and 5% $CO_2$ for 24 hours.
8) After freeze-drying, the liposome stored in the glass vial was hydrated with 225 μL of distilled water and allowed to react at 60°C for 10 minutes to thus completely dissolve the liposome (liposome concentration: 4.4 mg/mL).
9) 22.5 μL of the liposome, 10 μL of saponin (0.25 mg/mL), and 67.5 μL of a buffer were mixed in a 96-well plate and allowed to react at room temperature and 300 rpm for 30 minutes using an orbital shaker.
10) The mixture of liposome and saponin was dispensed at 20 μL/well into a 96-well plate and cultured at 37°C and 5% $CO_2$ for 18 hours.
11) Ez-Cytox and a cell culture medium were mixed at a ratio of 7:3, dispensed at 50 μL/well into a 24-well plate, and allowed to react at 37°C and 5% $CO_2$ for 3 hours.
12) The 96-well plate was centrifuged at 1500 rpm and room temperature for 5 minutes.
13) 100 μL of the supernatant was transferred to a new 96-well plate, and the absorbance was then measured at 450 nm.
14) Cytotoxicity (%) was calculated using the following equation.

$$\text{Cytotoxicity (\%)} = 100 - [(\text{OD of sample}/\text{OD of buffer}) \times 100]$$

Example 4-2: Results

As shown in FIG. 15, the higher the amount of the unsaturated fatty acid in the cationic liposome, the lower the cytotoxicity.

Based on the combination of the results thereof with the results of Example 3, it can be confirmed that the use of the cationic liposome containing the unsaturated fatty acid exhibited low cytotoxicity and was capable of 100% inhibiting hemolysis induced by saponin.

**Example 5: Screening for inhibition of hemolysis of various saponins**

The effect of the cationic liposome on inhibition of hemolysis induced by saponin was evaluated using various saponins, other than *Quillaja* saponaria-derived crude saponin and QS21.

Example 5-1: Method

Various liposomes were manufactured using a lipid film method, and hemolysis analysis was performed.

The types of saponin and cationic liposome used in this Example are shown in the following Table 12 and Table 13, respectively.

[Table 12]

| Saponin | | |
|---|---|---|
| Classification | Type | Amount (μg) |
| Steroidal saponin | Digitonin | 2.5 |
| | Paris VII | 1.25 |
| Triterpenoid saponin | Aescin | 2.5 |
| | a-Hederin | 1.25 |

From the amounts shown in Table 12, a saponin concentration causing 60% hemolysis was selected because there is a limit to the amount of liposome that may be used.

[Table 13]

| Cationic liposome | | | |
|---|---|---|---|
| Type | Lipid | | Amount (μg) |
| | Cationic | Neutral | |
| DDA:DMPC | 18:0 | 14:0 | 400, 200, 100, 50, 25, 13, 6, 0 |
| DDA:DOPC | 18:0 | 18:1 | |
| DOTAP:DMPC | 18:1 | 14:0 | 400, 200, 100, 50, 25, 13, 6, 0 |
| DOTAP:DOPC | 18:1 | 18:1 | |

Example 5-2: Manufacture of liposomes

The liposome used in this Example was manufactured using the following method.

1) Each of a cationic lipid and a neutral lipid was weighed and placed in a glass tube.

2) Chloroform was added thereto such that the lipid concentration was 4 mg/mL, and was completely dissolved at 37°C for 10 minutes to afford a lipid solution.

3) Each lipid mixture was prepared by mixing a cationic lipid solution and a neutral lipid solution at a weight ratio of 1:1 in a round-bottom flask.

4) Using a rotary evaporator, volatilization was performed for 30 minutes at 60°C for the lipid mixture containing DOTAP and at 80°C for the lipid mixture containing DDA, and thus whether chloroform did not remain and a lipid membrane film was formed on the wall of the flask was observed.

5) Sucrose in a HEPES buffer (pH 7.4) was placed in the flask such that the liposome concentration was 4 mg/mL, and the lipid membrane was dissolved at 60°C.

6) The liposome thus manufactured was stored in a refrigerator at 4°C until the test.

Example 5-3: Method of hemolysis analysis

1) The liposome, saponin, sucrose in a HEPES buffer (pH 7.4), and distilled water were placed in a 96-well plate, as shown in Tables 14 and 15 below.

[Table 14]

| **With saponin** (liposome concentration: 4 mg/mL, saponin concentration (digitonin: 0.25 mg/mL, Paris VII: 0.125 mg/mL, aescin: 0.25 mg/mL, α-hederin: 0.125 mg/mL)) | | | | | | | | | | | |
|---|---|---|---|---|---|---|---|---|---|---|---|
| **Group** | **Liposome conc. (pg/well)** | | | | | | | | | **100% Hemolys is control** | **0% Hemolys is control** |
| | **0** | **3** | **6** | **13** | **25** | **50** | **100** | **200** | **400** | | |
| Liposome (μl) | 0.0 | 0.8 | 1.6 | 3.1 | 6.3 | 12. 5 | 25. 0 | 50. 0 | 100. 0 | 0 | 0 |
| Saponin (μl) | 10.0 | 10. 0 | 10. 0 | 10. 0 | 10. 0 | 10. 0 | 10. 0 | 10. 0 | 10.0 | 0 | 0 |
| Buffer (μl) | 100. 0 | 99. 2 | 98. 4 | 96. 9 | 93. 8 | 87. 5 | 75. 0 | 50. 0 | 0.0 | 0 | 110 |
| D.W. (μl) | 0.0 | 0.0 | 0.0 | 0.0 | 0.0 | 0.0 | 0.0 | 0.0 | 0.0 | 110 | 0 |

[Table 15]

| **Without saponin** | | | | | | | | | | | |
|---|---|---|---|---|---|---|---|---|---|---|---|
| **Group** | **Liposome conc. (pg/well)** | | | | | | | | | **100% Hemolysis control** | **0% Hemolysis control** |
| | **0** | **3** | **6** | **13** | **25** | **50** | **100** | **200** | **400** | | |
| Liposome (μl) | 0.0 | 0.8 | 1.6 | 3.1 | 6.3 | 12. 5 | 25. 0 | 50. 0 | 22. 5 | 0 | 0 |
| Saponin (μl) | 0.0 | 0.0 | 0.0 | 0.0 | 0.0 | 0.0 | 0.0 | 0.0 | 0.0 | 0 | 0 |
| Buffer (μl) | 100. 0 | 99. 2 | 98. 4 | 96. 9 | 93. 8 | 87. 5 | 75. 0 | 50. 0 | 77. 5 | 0 | 110 |
| D.W. (μl) | 10.0 | 10. 0 | 10. 0 | 10. 0 | 10. 0 | 10. 0 | 10. 0 | 10. 0 | 10. 0 | 110 | 0 |

2) The reaction was carried out at room temperature and 300 rpm for 30 minutes using an orbital shaker.
3) 3 mL of RBCs were suspended in 10 mL of PBS and centrifuged at room temperature and 1500 rpm for 5 minutes, after which the supernatant was removed.
4) Steps 2) and 3) were repeated three times, and thus the RBCs were washed.
5) The RBC pellet was suspended in 1 mL of PBS and diluted to 1/100, followed by cell counting.
6) The RBCs were diluted to $1.5\times10^9$ cells/mL using PBS and dispensed at $3\times10^7$ cells/20 μL/well in a 96-well plate treated with the test material of 2) above.

7) The reaction was carried out at room temperature and 300 rpm for 1 hour using an orbital shaker.
8) The 96-well plate was centrifuged at room temperature and 1500 rpm for 5 minutes.
9) 50 μL of the supernatant was transferred to a new 96-well plate, and the absorbance was then measured at 415 nm.
10) Hemolysis (%) was calculated using the following equation.

$$\text{Hemolysis (\%)} = (\text{OD of sample} - \text{OD of 0\% hemolysis})/(\text{OD of 100\% hemolysis} - \text{OD of 0\% hemolysis}) \times 100$$

Example 5-4: Confirmation of effect of inhibition of hemolysis induced by steroidal saponin

Based on the results of measurement of the concentration of digitonin or Paris VII causing 100% hemolysis, 100% hemolysis was observed when the amount of digitonin was 10 μg or when the amount of Paris VII was 5 μg, and 60% hemolysis was observed when the amount of digitonin was 2.5 μg or when the amount of Paris VII was 1.25 μg (FIG. 16).

Accordingly, based on the results of measurement of the concentrations of DDA(18:0):DMPC(14:0) and DDA(18:0):DOPC(18:1) that inhibit the hemolysis induced by 2.5 μg of digitonin, DDA:DMPC did not inhibit hemolysis induced by digitonin, and hemolysis induced by digitonin was 100% inhibited when the amount of DDA:DOPC was 400 μg (liposome:saponin = 160:1) (FIG. 17).

Based on the results of measurement of the concentrations of DOTAP(18:1):DMPC(14:0) and DOTAP(18:1):DOPC(18:1) that inhibit the hemolysis induced by 2.5 μg of digitonin, hemolysis induced by digitonin was 100% inhibited when the amount of DOTAP:DMPC was 400 μg (liposome:saponin = 160:1), and hemolysis induced by digitonin was 100% inhibited when the amount of DOTAP:DOPC was 200 μg (liposome:saponin = 80:1) (FIG. 18).

In addition, based on the results of measurement of the concentrations of DDA(18:0):DMPC(14:0) and DDA(18:0):DOPC(18:1) that inhibit the hemolysis induced by 1.25 μg of Paris VII, DDA:DMPC did not inhibit hemolysis induced by Paris VII, and hemolysis induced by Paris VII was 50% inhibited when the amount of DDA:DOPC was 400 μg (FIG. 19) .

Based on the results of measurement of the concentrations of DOTAP(18:1):DMPC(14:0) and DOTAP(18:1):DOPC(18:1) that inhibit the hemolysis induced by 1.25 μg of Paris VII, hemolysis induced by Paris VII was 100% inhibited when the amount of DOTAP:DMPC was 400 μg (liposome:saponin = 320:1), and hemolysis induced by Paris VII was 100% inhibited when the amount of DOTAP:DOPC was 400 μg (FIG. 20).

Example 5-5: Confirmation of effect of inhibition of hemolysis induced by triterpenoid saponin

Based on the results of measurement of the concentration of aescin or α-hederin causing 100% hemolysis, 100% hemolysis was observed when the amount of aescin was 5 μg or when the amount of α-hederin was 10 μg, and 60% hemolysis was observed when the amount of aescin was 2.5 μg or when the amount of α-hederin was 1.25 μg (FIG. 21).

Accordingly, based on the results of measurement of the concentrations of DDA(18:0):DMPC(14:0) and DDA(18:0):DOPC(18:1) that inhibit the hemolysis induced by 2.5 μg of aescin, DDA:DMPC did not inhibit hemolysis induced by aescin, and hemolysis induced by aescin was 100% inhibited when the amount of DDA:DOPC was 400 μg (liposome:saponin = 160:1) (FIG. 22).

Based on the results of measurement of the concentrations of DOTAP(18:1):DMPC(14:0) and DOTAP(18:1):DOPC(18:1) that inhibit the hemolysis induced by 2.5 μg of aescin, hemolysis induced by aescin was 100% inhibited when the amount of each of DOTAP:DMPC and DOTAP:DOPC was 200 μg (liposome:saponin = 80:1) (FIG. 23).

In addition, based on the results of measurement of the concentrations of DDA(18:0):DMPC(14:0) and DDA(18:0):DOPC(18:1) that inhibit the hemolysis induced by 1.25 μg of α-hederin, DDA:DMPC did not inhibit hemolysis induced by α-hederin, and hemolysis induced by α-hederin was 100% inhibited when the amount of DDA:DOPC was 13 μg (liposome:saponin = 10.4:1) (FIG. 24).

Based on the results of measurement of the concentrations of DOTAP(18:1):DMPC(14:0) and DOTAP(18:1):DOPC(18:1) that inhibit the hemolysis induced by 1.25 μg of α-hederin, hemolysis induced by α-hederin was 100% inhibited when the amount of each of DOTAP:DMPC and DOTAP:DOPC was 13 μg (liposome:saponin = 10.4:1) (FIG. 25).

Examples 5-6: Results

The amounts of liposomes inhibiting hemolysis induced by various saponins are shown in Table 16 below.

[Table 16]

| Liposome | | | Steroidal saponin | | | | Triterpenoid saponin | | | |
|---|---|---|---|---|---|---|---|---|---|---|
| Type | Lipid | | Digitonin | | Paris VII | | Aescin | | α-Hederi n | |
| | Cationi c | Neutra l | $IC_{50}$ | $IC_{100}$ | $IC_{50}$ | $IC_{100}$ | $IC_{50}$ | $IC_{100}$ | $IC_5$ 0 | $IC_{10}$ 0 |
| DDA:DMPC | 18:0 | 14:0 | - | - | - | - | - | - | - | - |
| DDA:DOPC | 18:0 | 18:1 | 198. 9 | 400. 0 | 256. 2 | - | 222. 5 | 400. 0 | 8.3 | 12. 5 |
| DOTAP:DMP C | 18:1 | 14:0 | 105. 4 | 400. 0 | 219. 8 | 400. 0 | 88.9 | 200. 0 | 7.5 | 12. 5 |
| DOTAP:DOP C | 18:1 | 18:1 | 64.4 | 200. 0 | 188. 3 | 400. 0 | 66.1 | 200. 0 | 6.1 | 12. 5 |
| $IC_{50}$: Liposome concentration that inhibits 50% of hemolysis.<br>$IC_{100}$: Liposome concentration that inhibits 100% of hemolysis. | | | | | | | | | | |

As is apparent from the results described above, it can be confirmed that, when the cationic liposome contains the unsaturated fatty acid therein, the effect thereof on inhibition of hemolysis induced by saponin is excellent.

**Example 6: Confirmation of ratio of cationic and neutral lipids in cationic liposome that inhibits hemolysis induced by saponin**

In order to confirm the ratio of cationic lipid to neutral lipid in the cationic liposome that inhibits hemolysis induced by saponin, cationic liposomes were manufactured at various ratios of cationic lipid and neutral lipid using a freeze-drying method, and hemolysis analysis was performed.

Example 6-1: Manufacture of liposomes

The types of liposomes used in this Example are shown in Table 17 below.

[Table 17]

| No. | Liposome | Weight ratio (%) | Conc. (mg/mL) |
|---|---|---|---|
| 1 | DDA:DOPC | 0:100, 10:90, 20:80, 30:70, 40:60, 50:50, 60:40, 70:30, 80:20, 90:10, 100:0 | 2 |
| 2 | DOTAP:DMPC | | 2 |
| 3 | DOTAP:DOPC | | 2 |

The liposome shown in Table 17 was manufactured using the following method.
1) 240 mg of each of DDA, DOPC, DOTAP, and DMPC was weighed and placed in a 70 mL glass vial.
2) 60 mL of t-butyl alcohol was placed in each vial to prepare a 4 mg/mL lipid stock solution, which was then heated for 10 minutes to thus completely dissolve the lipid.
3) Each lipid mixture was prepared by mixing DDA, DOPC, DOTAP, and DMPC in a 10 mL glass vial, as shown in Table 18 below.

[Table 18]

| Weight ratio (%) | DDA:DOPC | | DOTAP:DMPC | | DOTAP:DOPC | |
|---|---|---|---|---|---|---|
| | DDA | DOPC | DOTAP | DMPC | DOTAP | DOPC |
| 0:100 | 0.0 | 5.0 | 0.0 | 5.0 | 0.0 | 5.0 |
| 10:90 | 0.5 | 4.5 | 0.5 | 4.5 | 0.5 | 4.5 |
| 20:80 | 1.0 | 4.0 | 1.0 | 4.0 | 1.0 | 4.0 |
| 30:70 | 1.5 | 3.5 | 1.5 | 3.5 | 1.5 | 3.5 |

(continued)

| Weight ratio (%) | DDA:DOPC | | DOTAP:DMPC | | DOTAP:DOPC | |
|---|---|---|---|---|---|---|
| | DDA | DOPC | DOTAP | DMPC | DOTAP | DOPC |
| 40:60 | 2.0 | 3.0 | 2.0 | 3.0 | 2.0 | 3.0 |
| 50:50 | 2.5 | 2.5 | 2.5 | 2.5 | 2.5 | 2.5 |
| 60:40 | 3.0 | 2.0 | 3.0 | 2.0 | 3.0 | 2.0 |
| 70:30 | 3.5 | 1.5 | 3.5 | 1.5 | 3.5 | 1.5 |
| 80:20 | 4.0 | 1.0 | 4.0 | 1.0 | 4.0 | 1.0 |
| 90:10 | 4.5 | 0.5 | 4.5 | 0.5 | 4.5 | 0.5 |
| 100:0 | 5.0 | 0.0 | 5.0 | 0.0 | 5.0 | 0.0 |

4) The inlet of each vial was covered with sealing tape, after which vortex mixing was performed for 5 seconds, and dense holes were formed in the sealing tape using a syringe needle.
5) After freezing in a freezer at -70°C for 2 hours, the resulting mixture was transferred to a freeze dryer, followed by freeze-drying at 20 Pa and -80°C for about 18 hours, thus volatilizing the organic solvent.
6) The resulting liposome cake was kept refrigerated until use and hydrated with 10 mL of sucrose in a HEPES buffer (pH 7.4) per vial for use in the experiment.

Example 6-2: Method of hemolysis analysis

1) The liposome, saponin, sucrose in a HEPES buffer (pH 7.4), and distilled water were placed in a 96-well plate, as shown in Tables 19 and 20 below.

[Table 19]

| **With saponin** (liposome concentration: 4 mg/mL, saponin concentration (digitonin: 0.25 mg/mL, α-hederin: 0.125 mg/mL, crude saponin: 0.25 mg/mL, QS21: 0.25 mg/mL)) | | | | | | | | | | | | | |
|---|---|---|---|---|---|---|---|---|---|---|---|---|---|
| **Group** | **Liposome (cationic lipid:neutral lipid)** | | | | | | | | | | | **100% Hemoly sis contro 1** | **0% Hemoly sis contro 1** |
| | **0:1 00** | **10: 90** | **20: 80** | **30: 70** | **40: 60** | **50: 50** | **60: 40** | **70: 30** | **80: 20** | **90: 10** | **100 :0** | | |
| Liposom e (μl) | 100 .0 | 100 .0 | 100 .0 | 100 .0 | 100 .0 | 100 .0 | 100 .0 | 100 .0 | 100 .0 | 100 .0 | 100 .0 | 0 | 0 |
| Saponin (μl) | 10. 0 | 10. 0 | 10. 0 | 10. 0 | 10. 0 | 10. 0 | 10. 0 | 10. 0 | 10. 0 | 10. 0 | 10. 0 | 0 | 0 |
| Buffer (μl) | 0.0 | 0.0 | 0.0 | 0.0 | 0.0 | 0.0 | 0.0 | 0.0 | 0.0 | 0.0 | 0.0 | 0 | 110 |
| D.W. (μl) | 0.0 | 0.0 | 0.0 | 0.0 | 0.0 | 0.0 | 0.0 | 0.0 | 0.0 | 0.0 | 0.0 | 110 | 0 |

[Table 20]

| Without saponin | | | | | | | | | | | | | |
|---|---|---|---|---|---|---|---|---|---|---|---|---|---|
| Group | Liposome (cationic lipid:neutral lipid) | | | | | | | | | | | 100% Hemolys is control | 0% Hemolys is control |
| | 0 : 100 | 10: 90 | 20: 80 | 30: 70 | 40: 60 | 50: 50 | 60: 40 | 70: 30 | 80: 20 | 90: 10 | 1000 | | |
| Liposo me (μl) | 100 0 | 100 0 | 100 0 | 100 0 | 100 0 | 100 0 | 100 0 | 100 0 | 100 0 | 100 0 | 100 0 | 0 | 0 |
| Saponi n (μl) | 0.0 | 0.0 | 0.0 | 0.0 | 0.0 | 0.0 | 0.0 | 0.0 | 0.0 | 0.0 | 0.0 | 0 | 0 |
| Buffer (μl) | 0.0 | 0.0 | 0.0 | 0.0 | 0.0 | 0.0 | 0.0 | 0.0 | 0.0 | 0.0 | 0.0 | 0 | 110 |
| D.W. (μl) | 10. 0 | 10. 0 | 10. 0 | 10. 0 | 10. 0 | 10. 0 | 10. 0 | 10. 0 | 10. 0 | 10. 0 | 10. 0 | 110 | 0 |

2) The reaction was carried out at room temperature and 300 rpm for 30 minutes using an orbital shaker.

3) 3 mL of RBCs were suspended in 10 mL of PBS and centrifuged at room temperature and 1500 rpm for 5 minutes, after which the supernatant was removed.

4) Steps 2) and 3) were repeated three times, and thus the RBCs were washed.

5) The RBC pellet was suspended in 1 mL of PBS and diluted to 1/100, followed by cell counting.

6) The RBCs were diluted to $1.5\times10^9$ cells/mL using PBS and dispensed at $3\times10^7$ cells/20 μL/well in a 96-well plate treated with the test material of 2) above.

7) The reaction was carried out at room temperature and 300 rpm for 1 hour using an orbital shaker.

8) The 96-well plate was centrifuged at room temperature and 1500 rpm for 5 minutes.

9) 50 μL of the supernatant was transferred to a new 96-well plate, and the absorbance was then measured at 415 nm.

10) Hemolysis (%) was calculated using the following equation.

$$\text{Hemolysis (\%)} = (\text{OD of sample} - \text{OD of 0\% hemolysis})/(\text{OD of 100\% hemolysis} - \text{OD of 0\% hemolysis}) \times 100$$

Example 6-3: Confirmation of ratio of cationic and neutral lipids in cationic liposome based on results of analysis of hemolysis

1) Lipid ratio of DDA(18:0):DOPC(18:1) that inhibits hemolysis of saponin

Hemolysis induced by 2.5 μg of digitonin was 100% inhibited when the amount of DDA in DDA:DOPC was 40% to 60%, and hemolysis induced by 1.25 μg of α-hederin was 100% inhibited when the amount of DDA in DDA:DOPC was 30% to 70% (FIG. 26).

Hemolysis induced by 2.5 μg of crude saponin was 100% inhibited when the amount of DDA in DDA:DOPC was 30% to 70%, and hemolysis induced by 2.5 μg of QS21 was 100% inhibited when the amount of DDA in DDA:DOPC was 30% to 70% (FIG. 27).

2) Lipid ratio of DOTAP(18:1):DMPC(14:0) that inhibits hemolysis of saponin

Hemolysis induced by 2.5 μg of digitonin was 100% inhibited when the amount of DOTAP in DOTAP:DMPC was 40% or more, and hemolysis induced by 1.25 μg of α-hederin was 100% inhibited when the amount of DOTAP in DOTAP:DMPC was 40% to 90% (FIG. 28).

Hemolysis induced by 2.5 μg of crude saponin was 100% inhibited when the amount of DOTAP in DOTAP:DMPC was 30% or more, and hemolysis induced by 2.5 μg of QS21 was 100% inhibited when the amount of DOTAP in DOTAP:DMPC was 30% or more (FIG. 29).

3) Lipid ratio of DOTAP(18:1):DOPC(18:1) that inhibits hemolysis of saponin

Hemolysis induced by 2.5 μg of digitonin was 100% inhibited when the amount of DOTAP in DOTAP:DOPC was 40% or more, and hemolysis induced by 1.25 μg of α-hederin was 100% inhibited when the amount of DOTAP in DOTAP:DOPC was 20% or more (FIG. 30).

Hemolysis induced by 2.5 μg of crude saponin was 100% inhibited when the amount of DOTAP in DOTAP:DOPC was 10% or more, and hemolysis induced by 2.5 μg of QS21 was 100% inhibited when the amount of DOTAP in DOTAP:DOPC was 10% or more (FIG. 31).

Example 6-4: Results

As described in Example 6-3, based on the results of measurement of the ratios of cationic lipids and neutral lipids in various cationic liposomes for various saponins, when the ratio of the cationic lipid in the cationic liposome was 10 to 100%, hemolysis induced by saponin was effectively inhibited (Table 21).

[Table 21]

| Ratio of cationic lipid in cationic liposome that inhibits hemolysis induced by various saponins | | | | | | | | | | | | |
|---|---|---|---|---|---|---|---|---|---|---|---|---|
| **Saponin** | **Liposome** | **Ratio of cationic lipid in cationic liposome (%, weight ratio)** | | | | | | | | | | |
| | | **0** | **10** | **20** | **30** | **40** | **50** | **60** | **70** | **80** | **90** | **100** |
| Digiton in | DDA(18:0) : DOPC(18:1 ) | | | | | ○ | ○ | ○ | | | | |
| α-Hederin | | | | | ○ | ○ | ○ | ○ | ○ | | | |
| Crude saponin | | | | | ○ | ○ | ○ | ○ | ○ | | | |
| QS21 | | | | | ○ | ○ | ○ | ○ | ○ | | | |
| Digiton in | DOTAP (18: 1) :DMPC(14: 0) | | | | | ○ | ○ | ○ | ○ | ○ | ○ | ○ |
| α-Hederin | | | | | | ○ | ○ | ○ | ○ | ○ | ○ | |
| Crude saponin | | | | | ○ | ○ | ○ | ○ | ○ | ○ | ○ | ○ |
| QS21 | | | | | ○ | ○ | ○ | ○ | ○ | ○ | ○ | ○ |
| Digiton in | DOTAP (18: 1) :DOPC(18: 1) | | | | | ○ | ○ | ○ | ○ | ○ | ○ | ○ |
| α-Hederin | | | | ○ | ○ | ○ | ○ | ○ | ○ | ○ | ○ | ○ |
| Crude saponin | | | ○ | ○ | ○ | ○ | ○ | ○ | ○ | ○ | ○ | ○ |
| QS21 | | | ○ | ○ | ○ | ○ | ○ | ○ | ○ | ○ | ○ | ○ |
| **Saponin** | **Liposome** | **Ratio of cationic lipid in cationic** | | | | | | | | | | |
| | | liposome (%, molar ratio) | | | | | | | | | | |
| | | 0 | 14 | 28 | 39 | 50 | 60 | 69 | 78 | 86 | 93 | 100 |
| Digiton in | DDA(18:0) : DOPC(18:1 ) | | | | | ○ | ○ | ○ | | | | |
| α-Hederin | | | | | ○ | ○ | ○ | ○ | ○ | | | |
| Crude saponin | | | | | ○ | ○ | ○ | ○ | ○ | | | |
| QS21 | | | | | ○ | ○ | ○ | ○ | ○ | | | |
| Saponin | Liposome | Ratio of cationic lipid in cationic liposome (%, molar ratio) | | | | | | | | | | |
| | | 0 | 15 | 27 | 39 | 50 | 60 | 69 | 78 | 86 | 93 | 100 |
| Digiton in | DOTAP (18: 1) :DMPC(14: 0) | | | | | ○ | ○ | ○ | ○ | ○ | ○ | ○ |
| α-Hederin | | | | | | ○ | ○ | ○ | ○ | ○ | ○ | |
| Crude saponin | | | | | ○ | ○ | ○ | ○ | ○ | ○ | ○ | ○ |
| QS21 | | | | | ○ | ○ | ○ | ○ | ○ | ○ | ○ | ○ |
| Digiton in | DOTAP (18: 1) :DOPC(18: 1) | | | | | ○ | ○ | ○ | ○ | ○ | ○ | ○ |
| α-Hederin | | | | ○ | ○ | ○ | ○ | ○ | ○ | ○ | ○ | ○ |
| Crude saponin | | | ○ | ○ | ○ | ○ | ○ | ○ | ○ | ○ | ○ | ○ |
| QS21 | | | ○ | ○ | ○ | ○ | ○ | ○ | ○ | ○ | ○ | ○ |
| ○: Hemolysis induced by saponin is inhibited. | | | | | | | | | | | | |

## INDUSTRIAL APPLICABILITY

Saponin exhibits a wide range of pharmacological and biological activities, such as anti-inflammatory activity, etc., including strong and effective immunological activity, and thus is effectively used medically and pharmaceutically, but has a disadvantage of causing hemolysis to red blood cells. In general, saponin is used along with cholesterol, etc. to inhibit the hemolysis of saponin, but in the present invention, it is confirmed that red blood cell hemolysis by saponin can be inhibited using a cationic liposome, which is more effective and economical in inhibiting the hemolysis of saponin. Therefore, according to the present invention, saponin can be more usefully applied to the manufacture of immunity enhancers, drug delivery carriers, etc.

Although specific embodiments of the present invention have been disclosed in detail as described above, it will be obvious to those skilled in the art that the description is merely of preferable exemplary embodiments and is not to be construed as limiting the scope of the present invention. Therefore, the substantial scope of the present invention will be defined by the appended claims and equivalents thereof.

## Claims

1. A composition for inhibiting red blood cell hemolysis by saponin comprising a cationic liposome and saponin, wherein the cationic liposome contains a cationic lipid and a neutral lipid.

2. The composition according to claim 1, wherein the cationic lipid or the neutral lipid comprises at least one unsaturated fatty acid.

3. The composition according to claim 1, wherein the cationic lipid is selected from the group consisting of 1,2-dioleoyl-3-(trimethylammonium)propane (DOTAP), dimethyldioctadecylammonium bromide (DDA), 3β-[N-(N',N'-dimethylaminoethane)carbamoyl]cholesterol (DC-Chol), 1,2-dioleoyl-3-(dimethylammonium)propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0-18:1 Ethyl PC), 1,2-dioleoyl-sn-glycero-3-ethylphosphocholine (18:1 Ethyl PC), 1,2-distearoyl-sn-glycero-3-ethylphosphocholin (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholin (12:0 Ethyl PC), N1-[2-((1S)-1-[(3-aminopropyl)amino]-4-[di(3-amino-propyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TA), 1,2-dimyristoyl-3-trimethylammonium-propane (14:0 TAP), and N4-cholesteryl-spermine (GL67).

4. The composition according to claim 1, wherein the neutral lipid is selected from the group consisting of 1,2-dimyristoyl-sn-glycero-3-phosphatidylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphatidylethanolamine (PE), phosphatidylglycerol (PG), phosphoric acid (PA), and phosphatidylcholine (PC).

5. The composition according to claim 1, wherein the cationic lipid is 1,2-dioleoyl-3-(trimethylammonium)propane (DOTAP) or dimethyldioctadecylammonium bromide (DDA), and the neutral lipid is any one selected from the group consisting of 1,2-dimyristoyl-sn-glycero-3-phosphorylcholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), and 1,2-distearoyl-sn-glycero-3-phosphocholine (DSPC).

6. The composition according to claim 1, wherein a weight ratio (%) of the cationic lipid in the cationic liposome is 10 to 100%.

7. The composition according to claim 1, wherein the saponin is *Quillaja* saponaria-derived crude saponin, QS21, a fraction containing QS21, steroidal saponin, or triterpenoid saponin.

8. A composition for immunity enhancement comprising the composition according to any one of claims 1 to 7.

9. The composition according to claim 8, further comprising an adjuvant.

**10.** A composition for drug delivery comprising the composition according to any one of claims 1 to 7.

**11.** A drug delivery carrier comprising a cationic liposome, wherein the cationic liposome contains a cationic lipid and a neutral lipid.

**12.** The drug delivery carrier according to claim 11, wherein the cationic lipid or the neutral lipid comprises at least one unsaturated fatty acid.

**13.** A drug-carrier complex in which a drug is adsorbed to or encapsulated in a cationic liposome, wherein the cationic liposome contains a cationic lipid and a neutral lipid.

**14.** The drug-carrier complex according to claim 13, wherein the cationic lipid or the neutral lipid comprises at least one unsaturated fatty acid.

FIG. 1

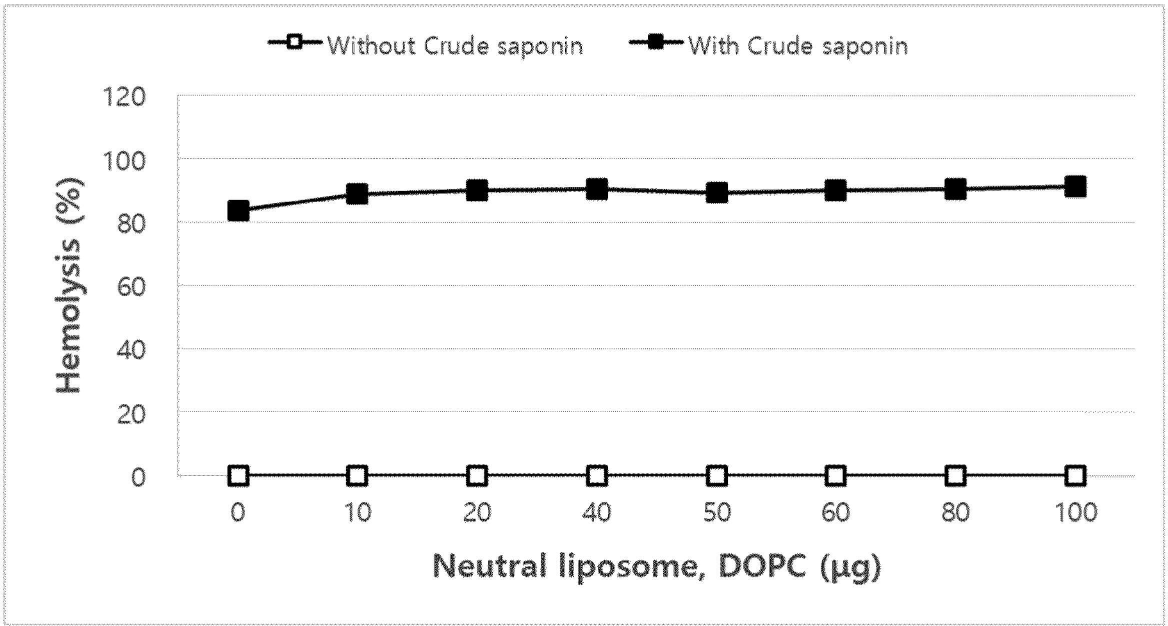
Without Crude saponin
With Crude saponin
Hemolysis (%)
120
100
80
60
40
20
0
0
10
20
40
50
60
80
100
Neutral liposome, DOPC (µg)

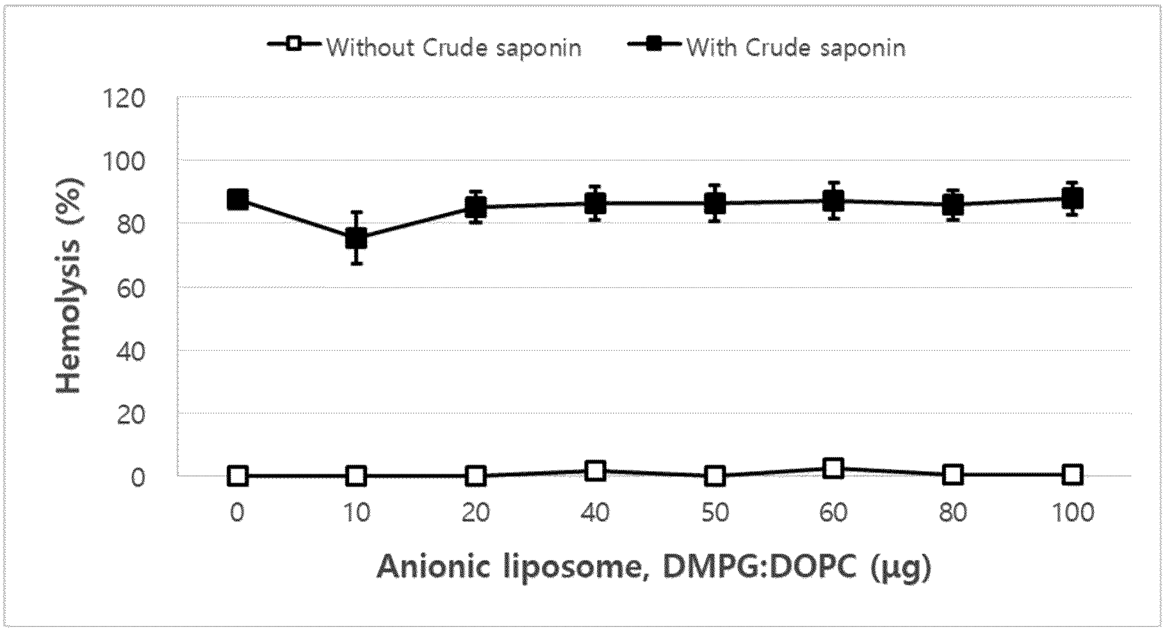
Without Crude saponin
With Crude saponin
Hemolysis (%)
120
100
80
60
40
20
0
0
10
20
40
50
60
80
100
Anionic liposome, DMPG:DOPC (µg)

FIG. 2

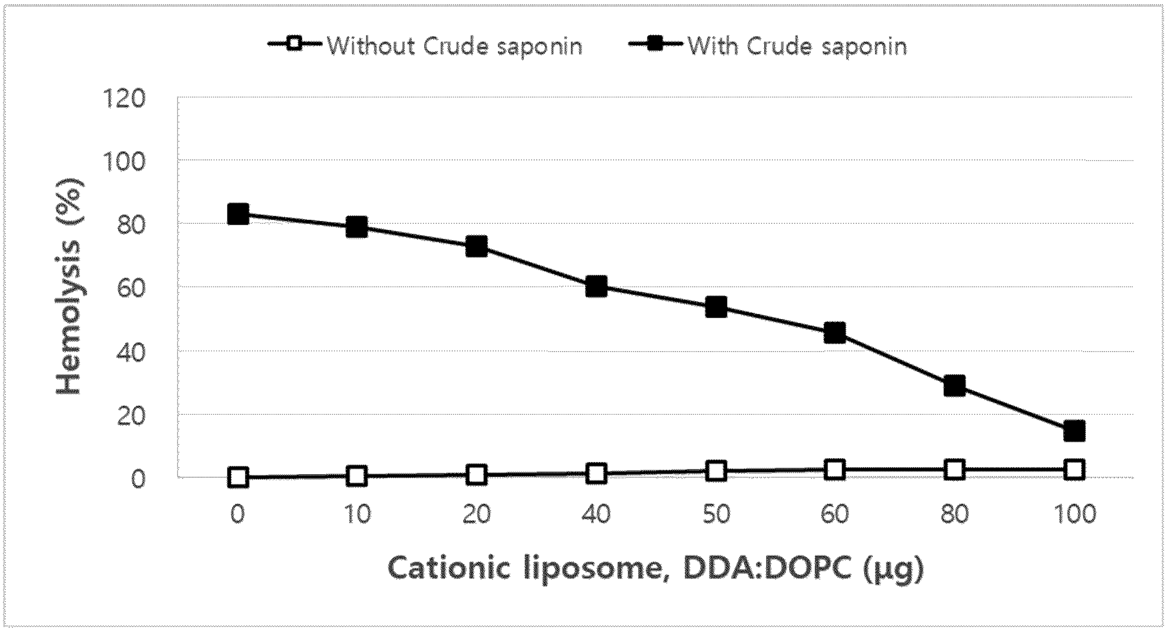
Without Crude saponin
With Crude saponin
Hemolysis (%)
120
100
80
60
40
20
0
0
10
20
40
50
60
80
100
Cationic liposome, DDA:DOPC (μg)

FIG. 3

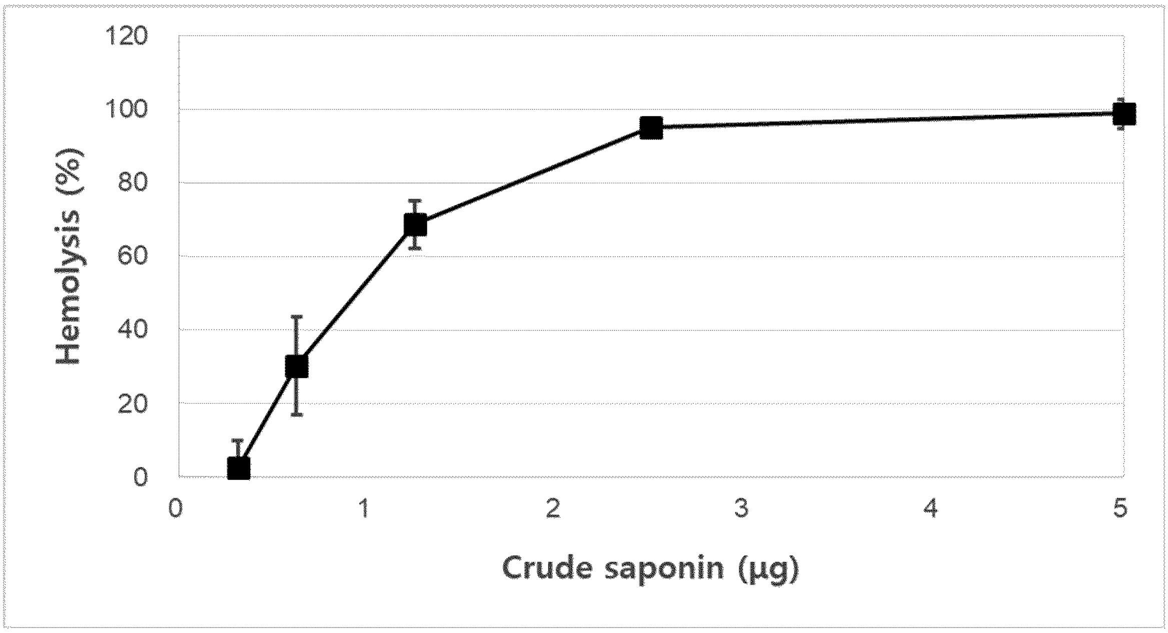
Hemolysis (%)
120
100
80
60
40
20
0
0
1
2
3
4
5
Crude saponin (μg)

FIG. 4

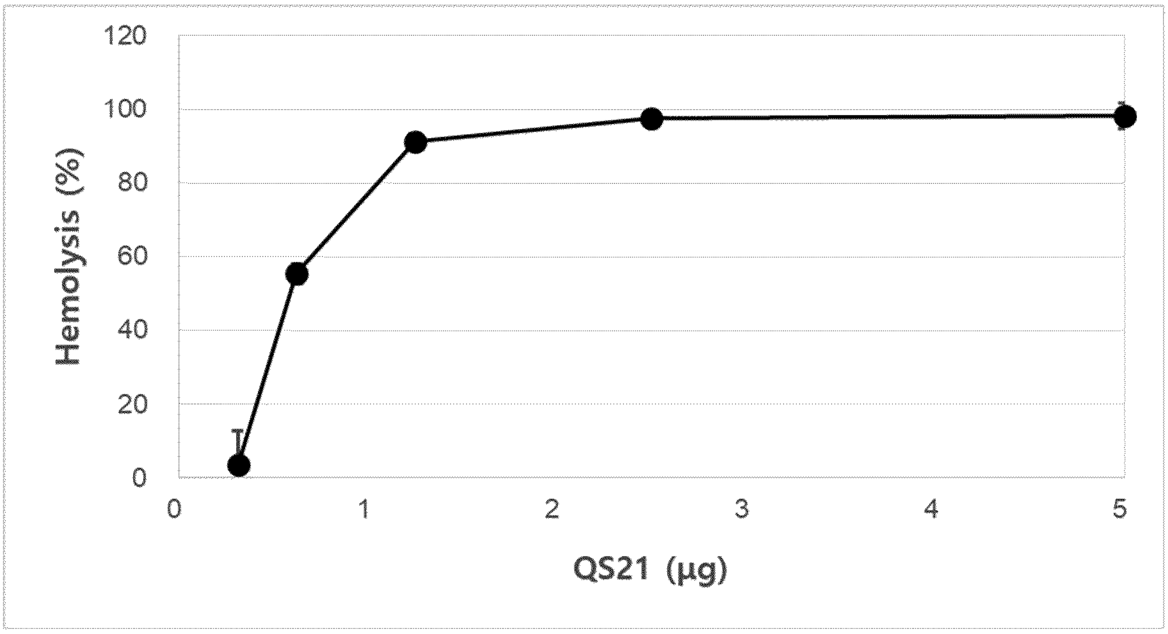

FIG. 5

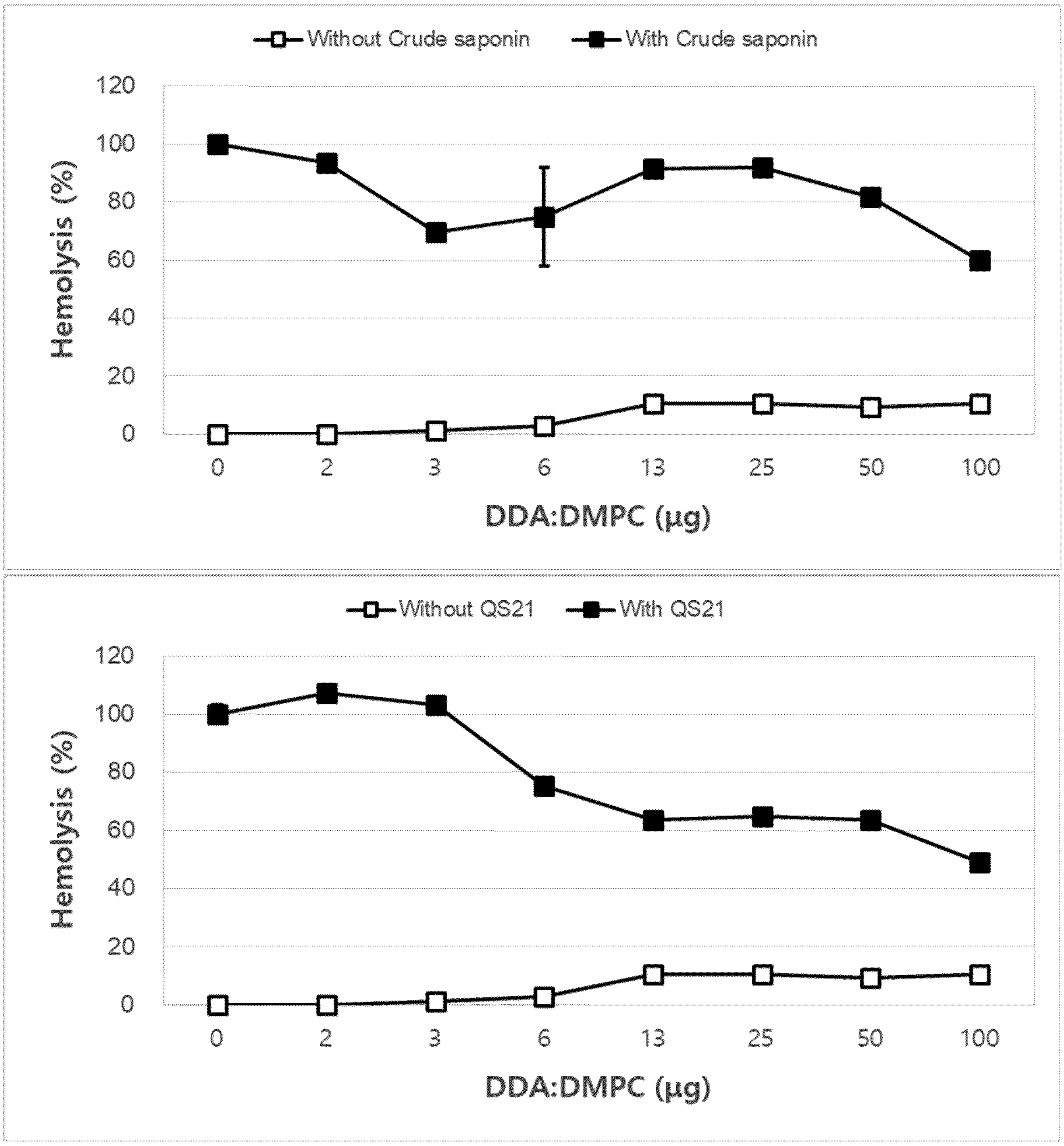
Without Crude saponin
With Crude saponin
Hemolysis (%)
120
100
80
60
40
20
0
0
2
3
6
13
25
50
100
DDA:DMPC (μg)
Without QS21
With QS21
Hemolysis (%)
120
100
80
60
40
20
0
0
2
3
6
13
25
50
100
DDA:DMPC (μg)

FIG. 6

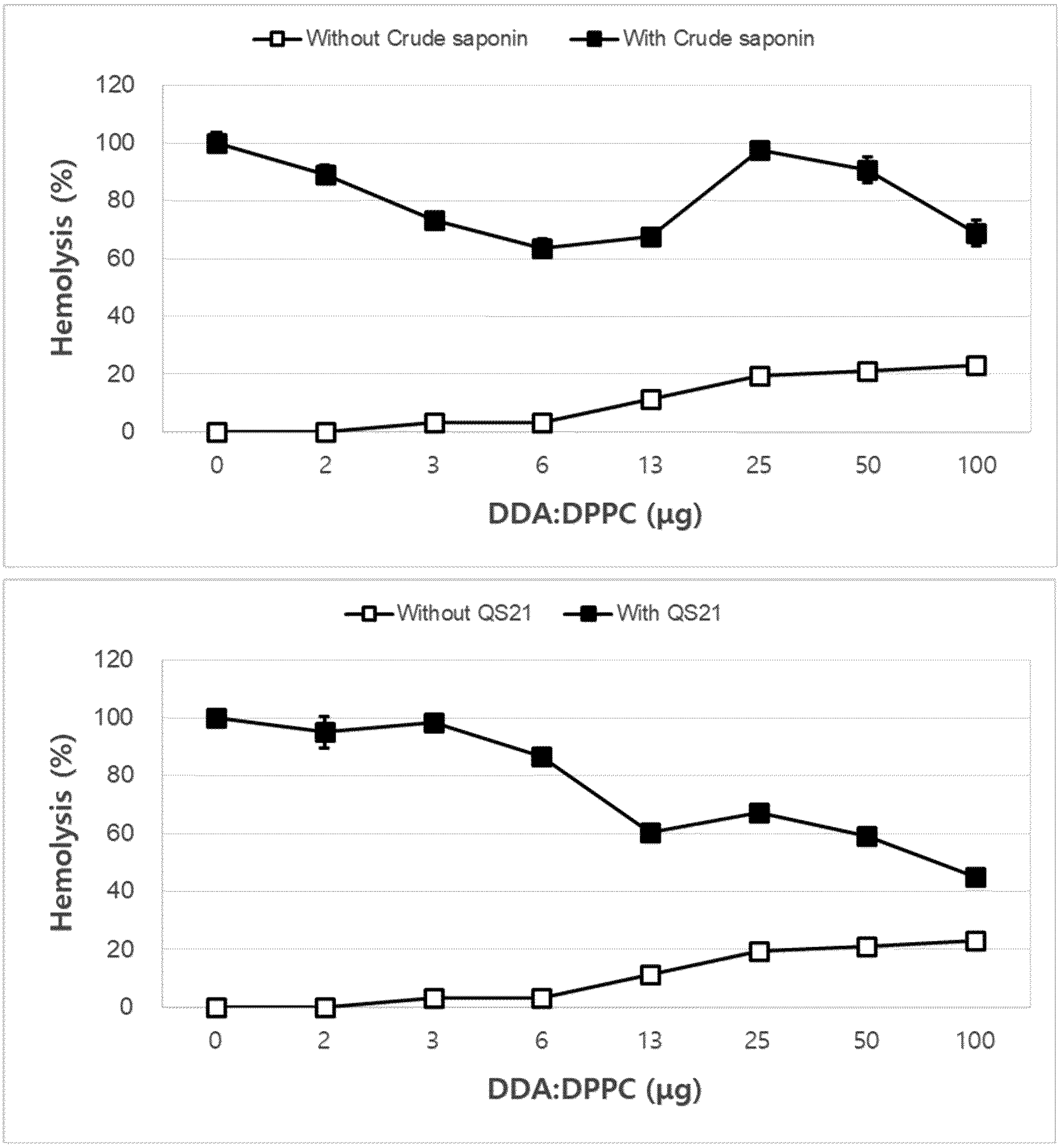
Without Crude saponin
With Crude saponin
Hemolysis (%)
120
100
80
60
40
20
0
0
2
3
6
13
25
50
100
DDA:DPPC (μg)
Without QS21
With QS21
Hemolysis (%)
120
100
80
60
40
20
0
0
2
3
6
13
25
50
100
DDA:DPPC (μg)

FIG. 7

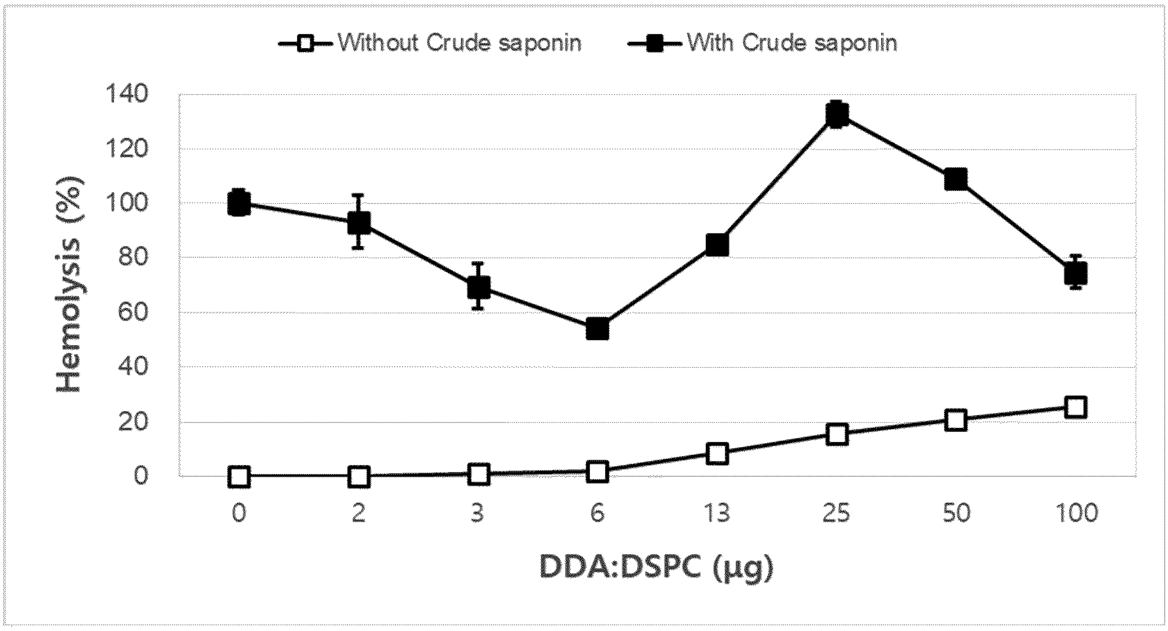
Without Crude saponin
With Crude saponin
Hemolysis (%)
140
120
100
80
60
40
20
0
0
2
3
6
13
25
50
100
DDA:DSPC (μg)

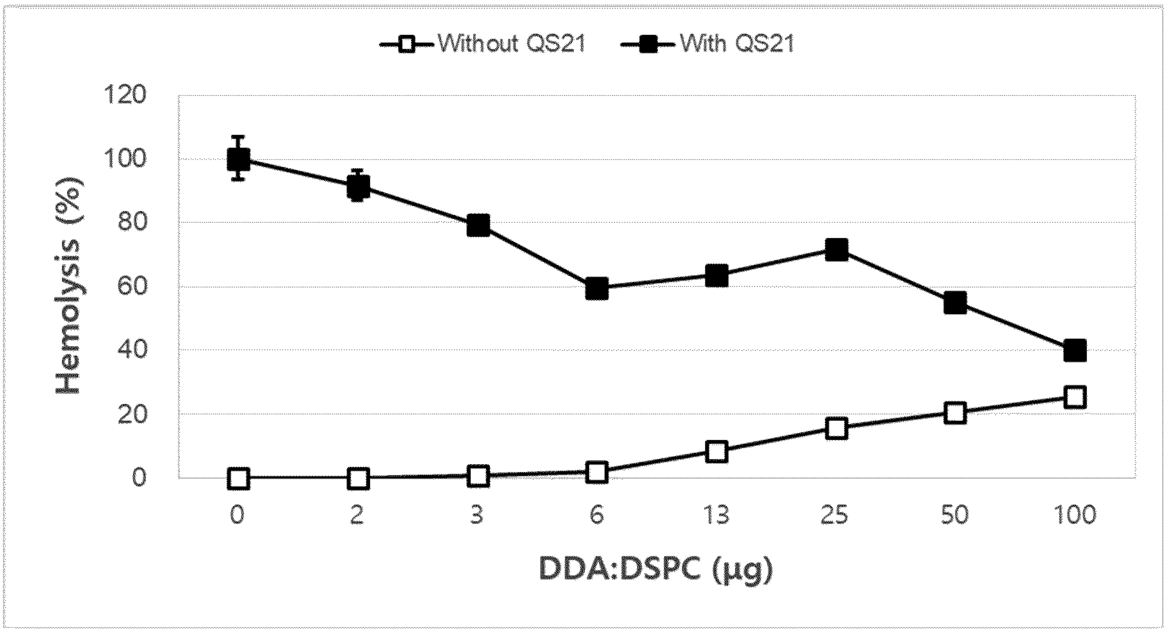
Without QS21
With QS21
Hemolysis (%)
120
100
80
60
40
20
0
0
2
3
6
13
25
50
100
DDA:DSPC (μg)

FIG. 8

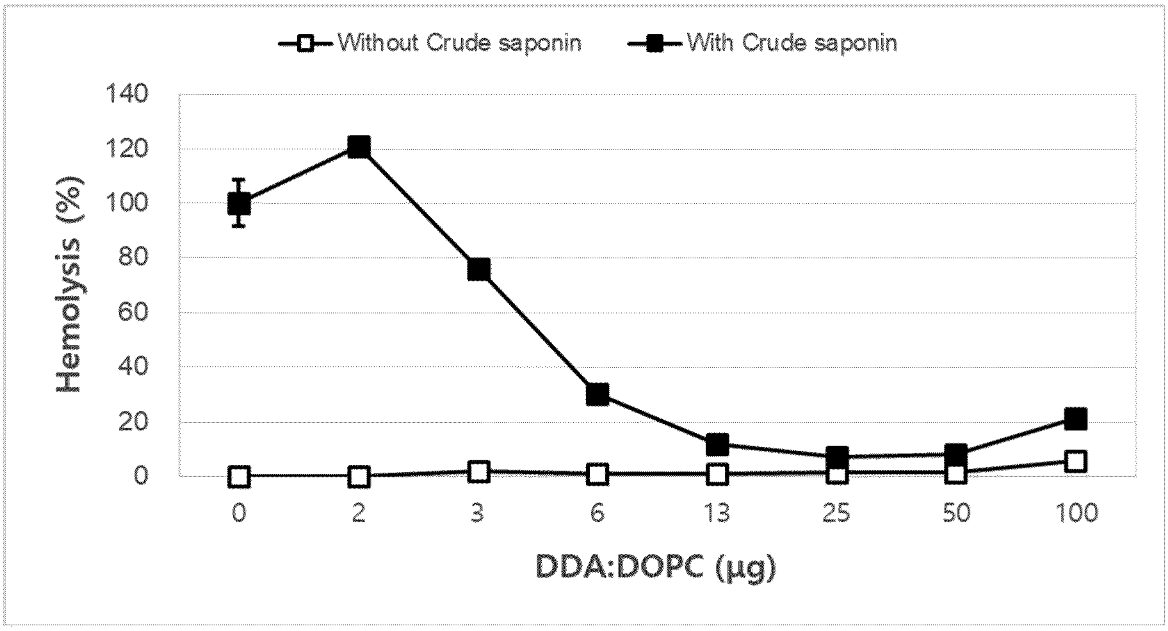
Without Crude saponin
With Crude saponin
Hemolysis (%)
140
120
100
80
60
40
20
0
0
2
3
6
13
25
50
100
DDA:DOPC (μg)

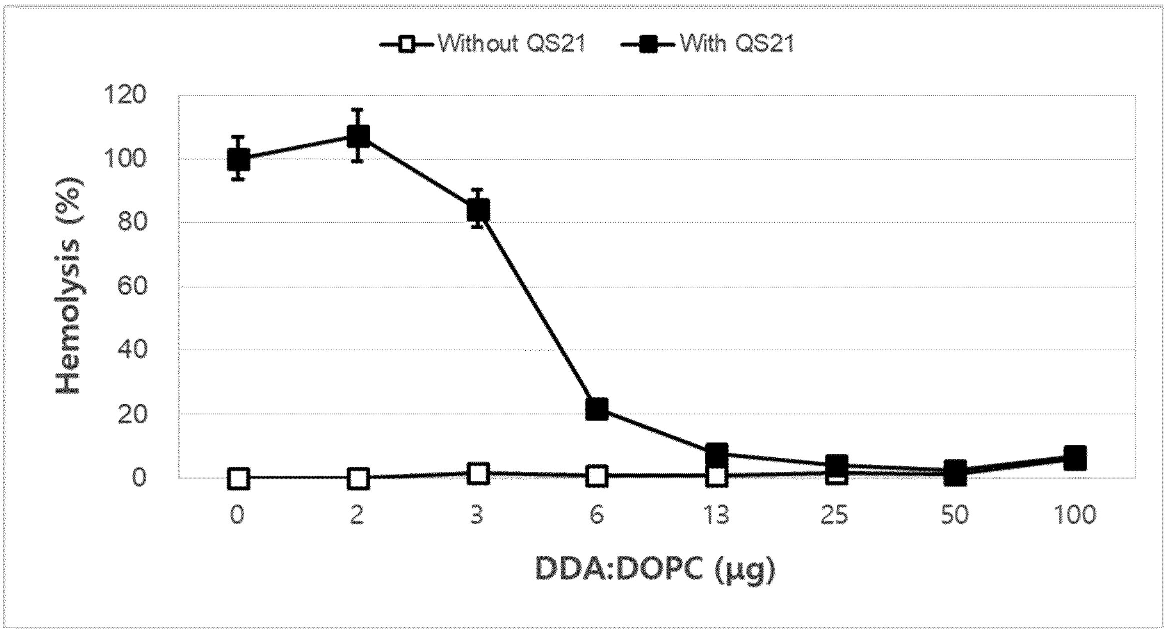
Without QS21
With QS21
Hemolysis (%)
120
100
80
60
40
20
0
0
2
3
6
13
25
50
100
DDA:DOPC (μg)

FIG. 9

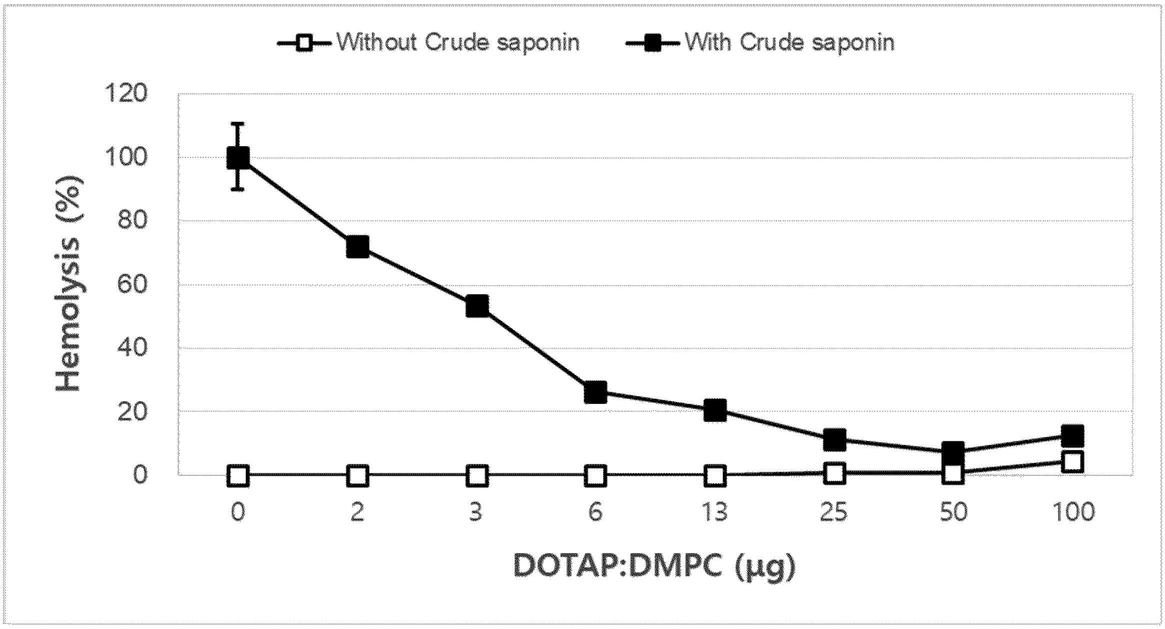
Without Crude saponin
With Crude saponin
Hemolysis (%)
120
100
80
60
40
20
0
0
2
3
6
13
25
50
100
DOTAP:DMPC (µg)

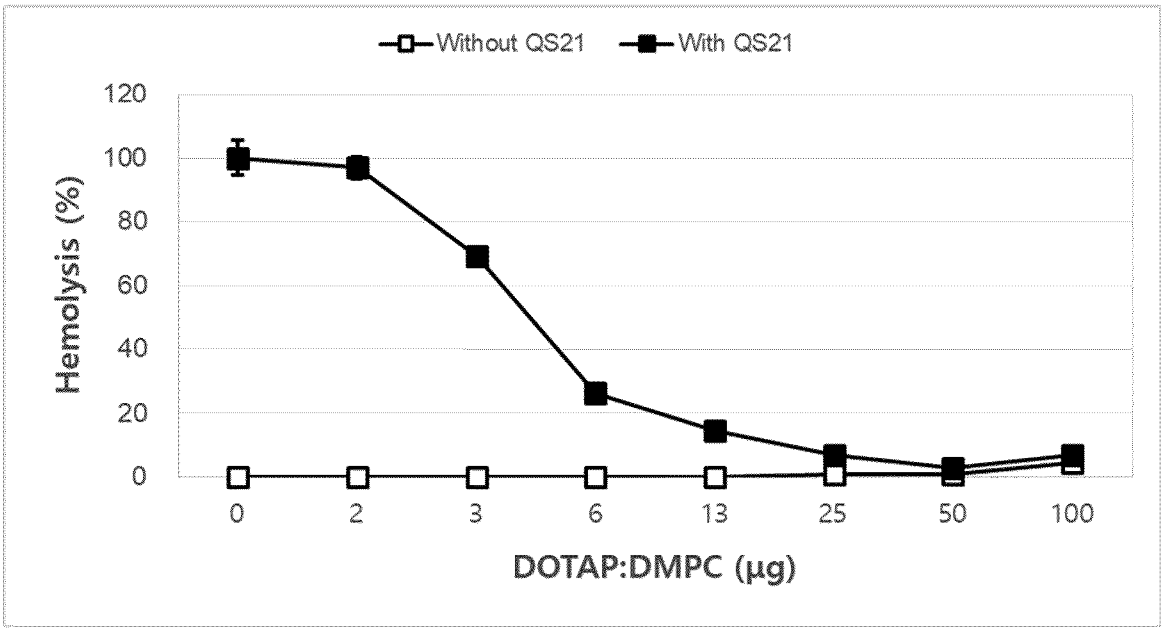
Without QS21
With QS21
Hemolysis (%)
120
100
80
60
40
20
0
0
2
3
6
13
25
50
100
DOTAP:DMPC (µg)

FIG. 10

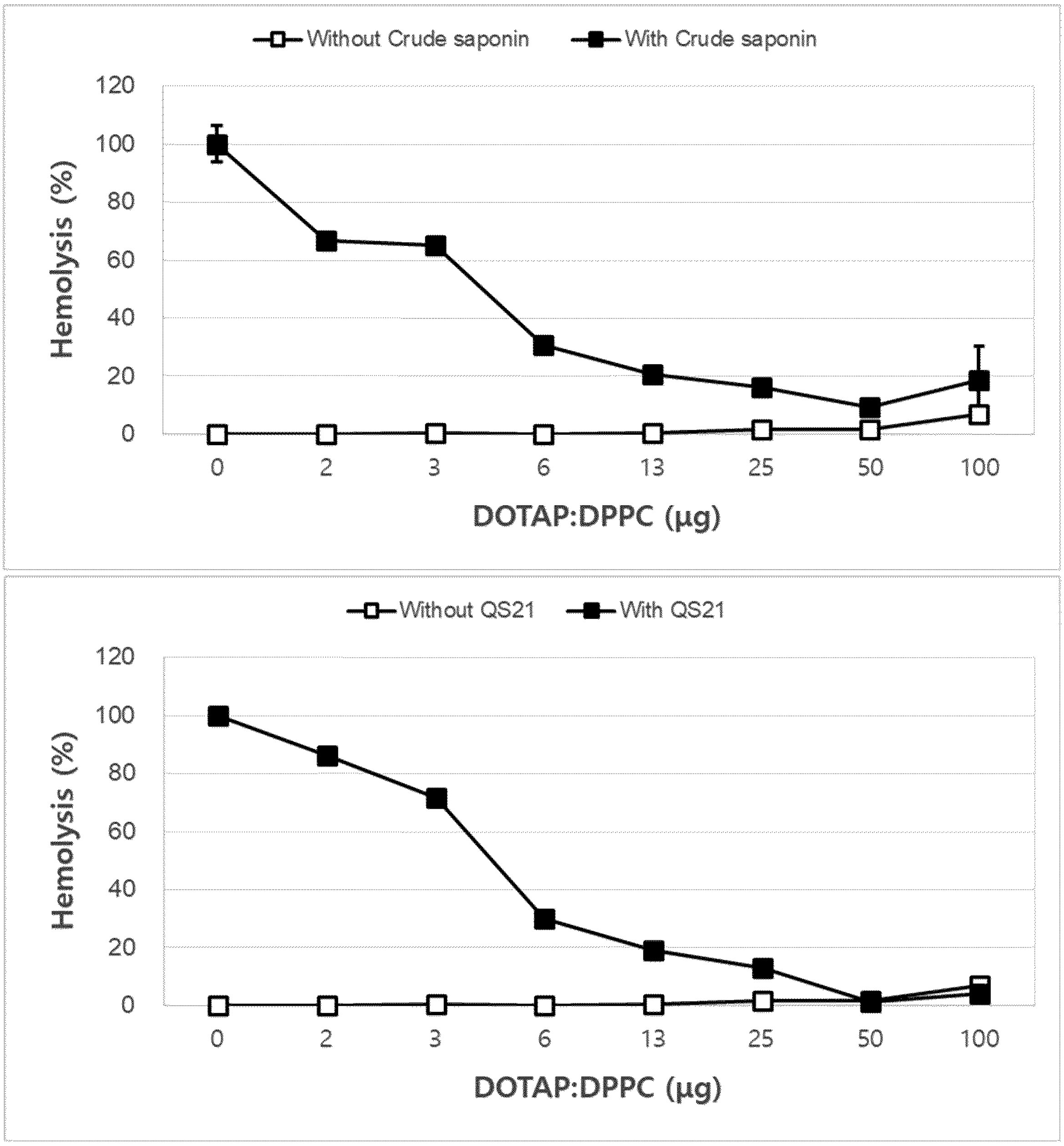
Without Crude saponin
With Crude saponin
Hemolysis (%)
120
100
80
60
40
20
0
0
2
3
6
13
25
50
100
DOTAP:DPPC (μg)
Without QS21
With QS21
Hemolysis (%)
120
100
80
60
40
20
0
0
2
3
6
13
25
50
100
DOTAP:DPPC (μg)

FIG. 11

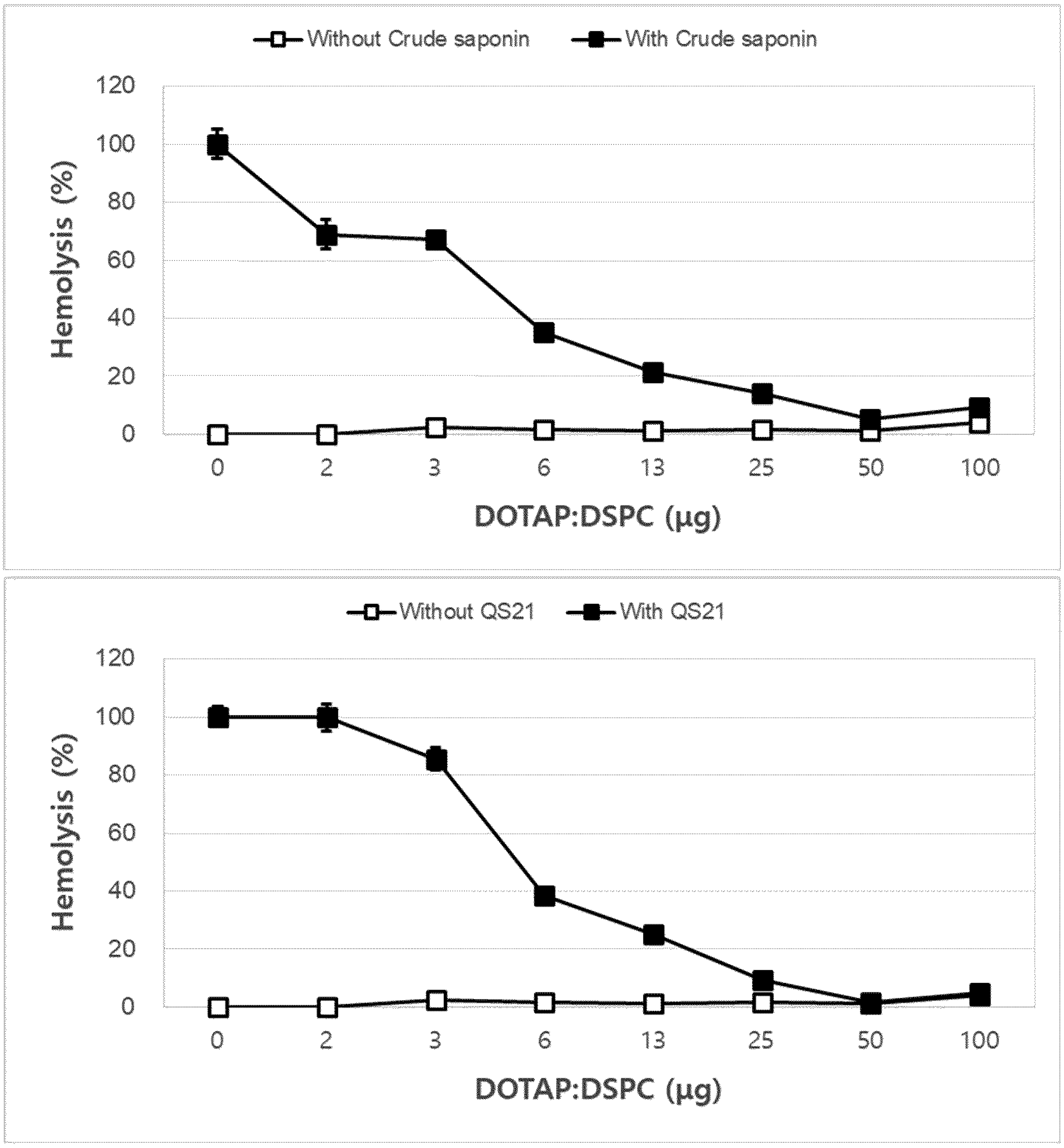
Without Crude saponin
With Crude saponin
Hemolysis (%)
120
100
80
60
40
20
0
0
2
3
6
13
25
50
100
DOTAP:DSPC (μg)
Without QS21
With QS21
Hemolysis (%)
120
100
80
60
40
20
0
0
2
3
6
13
25
50
100
DOTAP:DSPC (μg)

FIG. 12

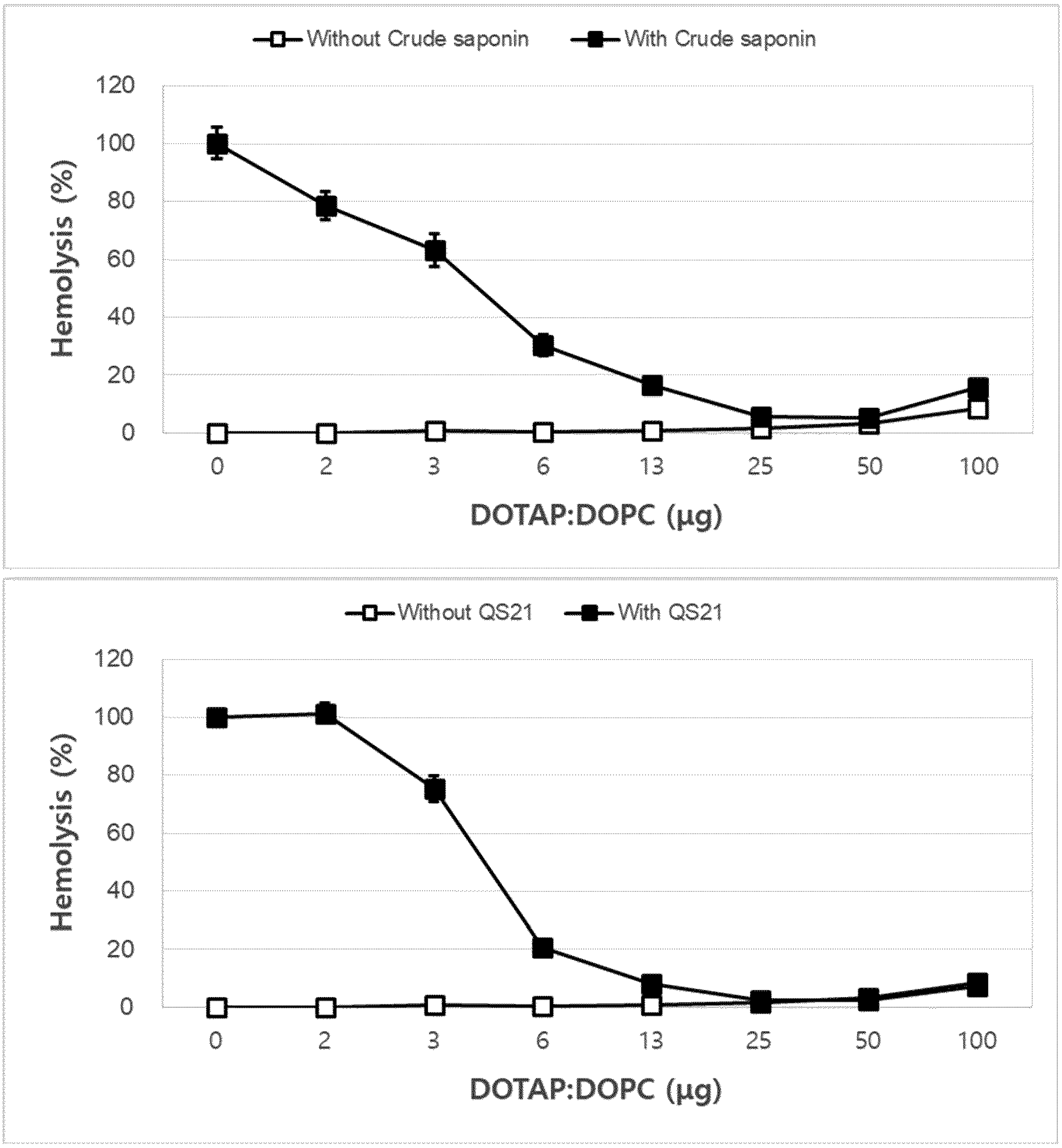
Without Crude saponin
With Crude saponin
Hemolysis (%)
120
100
80
60
40
20
0
0
2
3
6
13
25
50
100
DOTAP:DOPC (μg)
Without QS21
With QS21
Hemolysis (%)
120
100
80
60
40
20
0
0
2
3
6
13
25
50
100
DOTAP:DOPC (μg)

FIG. 13

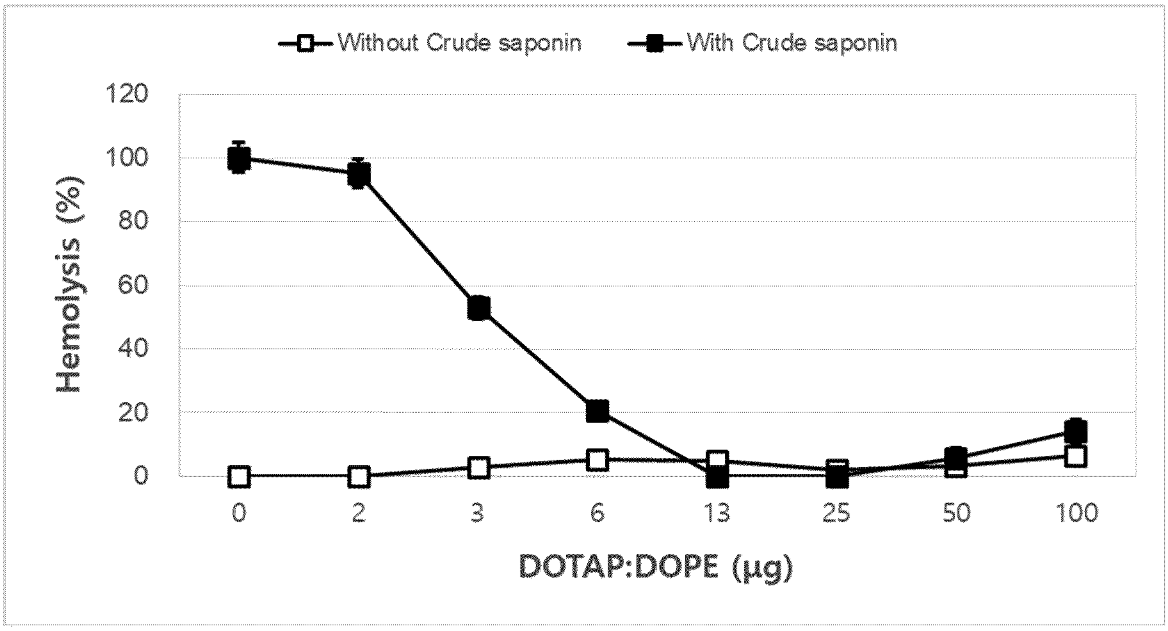
Without Crude saponin
With Crude saponin
Hemolysis (%)
120
100
80
60
40
20
0
0
2
3
6
13
25
50
100
DOTAP:DOPE (μg)

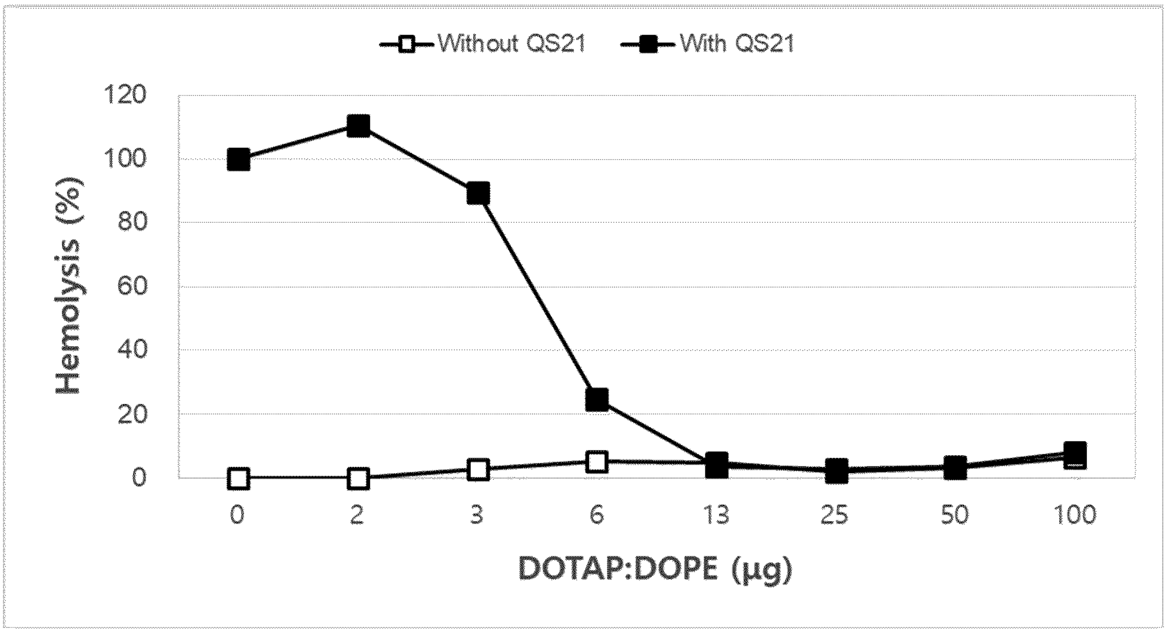
Without QS21
With QS21
Hemolysis (%)
120
100
80
60
40
20
0
0
2
3
6
13
25
50
100
DOTAP:DOPE (μg)

FIG. 14

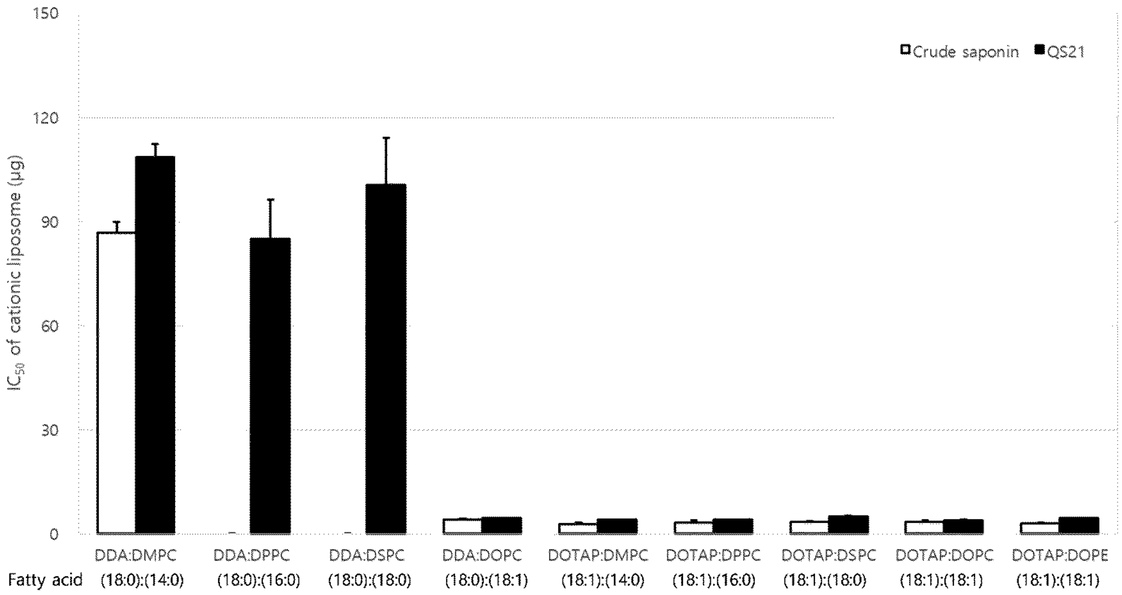
150
120
90
60
30
0
IC50 of cationic liposome (μg)
Crude saponin
QS21
DDA:DMPC
DDA:DPPC
DDA:DSPC
DDA:DOPC
DOTAP:DMPC
DOTAP:DPPC
DOTAP:DSPC
DOTAP:DOPC
DOTAP:DOPE
Fatty acid
(18:0):(14:0)
(18:0):(16:0)
(18:0):(18:0)
(18:0):(18:1)
(18:1):(14:0)
(18:1):(16:0)
(18:1):(18:0)
(18:1):(18:1)
(18:1):(18:1)

FIG. 15

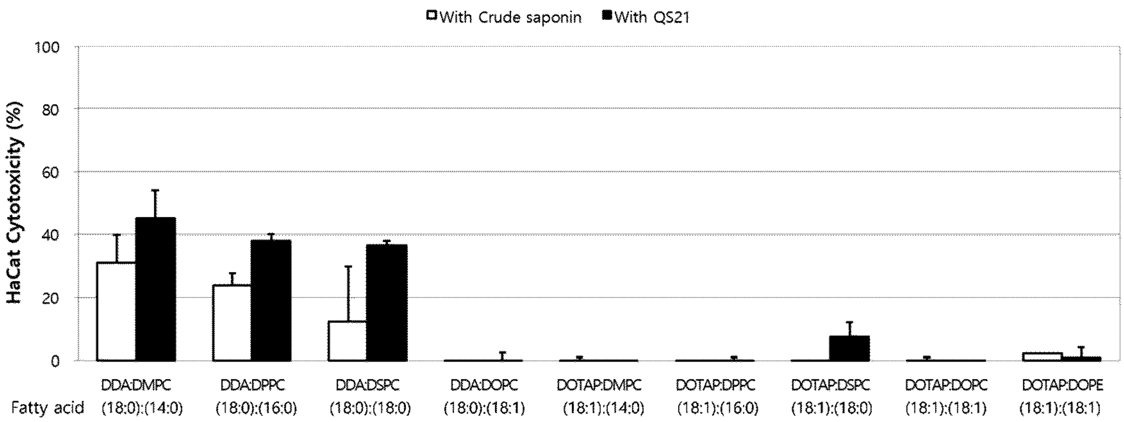
With Crude saponin
With QS21
100
80
60
40
20
0
HaCat Cytotoxicity (%)
DDA:DMPC
DDA:DPPC
DDA:DSPC
DDA:DOPC
DOTAP:DMPC
DOTAP:DPPC
DOTAP:DSPC
DOTAP:DOPC
DOTAP:DOPE
Fatty acid
(18:0):(14:0)
(18:0):(16:0)
(18:0):(18:0)
(18:0):(18:1)
(18:1):(14:0)
(18:1):(16:0)
(18:1):(18:0)
(18:1):(18:1)
(18:1):(18:1)

FIG. 16

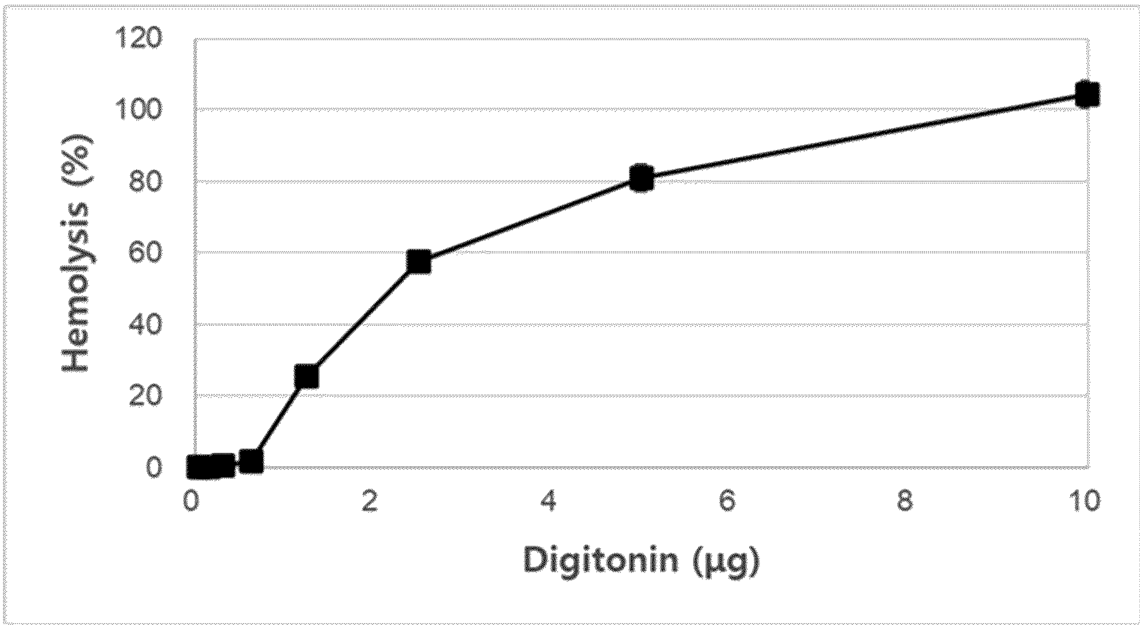
Hemolysis (%)
120
100
80
60
40
20
0
0
2
4
6
8
10
Digitonin (µg)

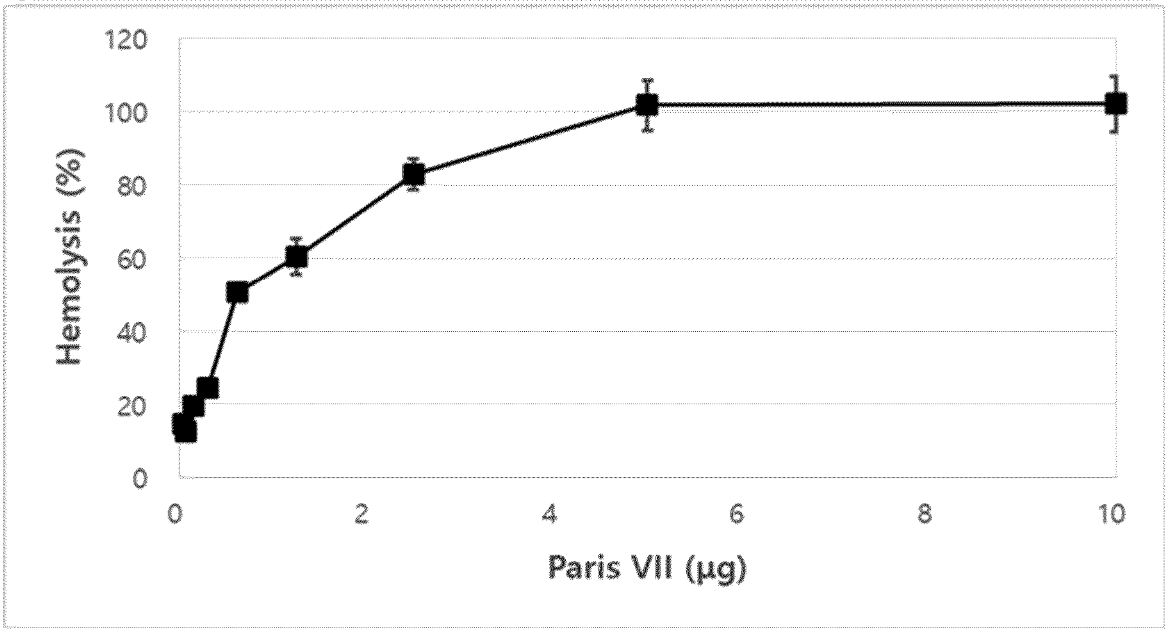
Hemolysis (%)
120
100
80
60
40
20
0
0
2
4
6
8
10
Paris VII (µg)

FIG. 17

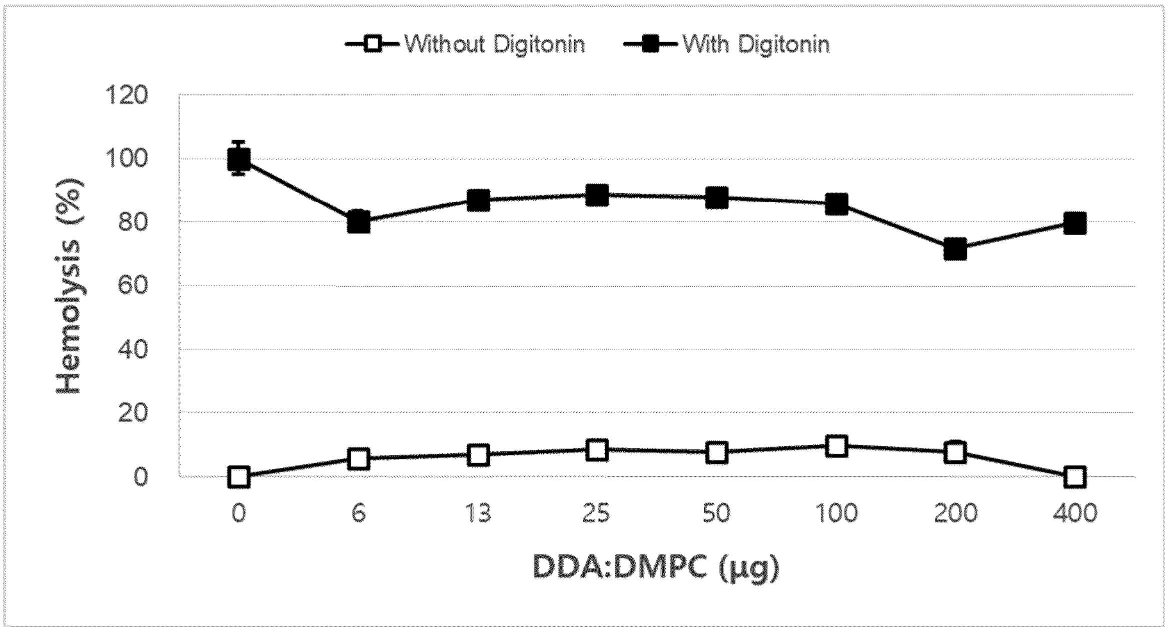
Without Digitonin
With Digitonin
Hemolysis (%)
120
100
80
60
40
20
0
0
6
13
25
50
100
200
400
DDA:DMPC (μg)

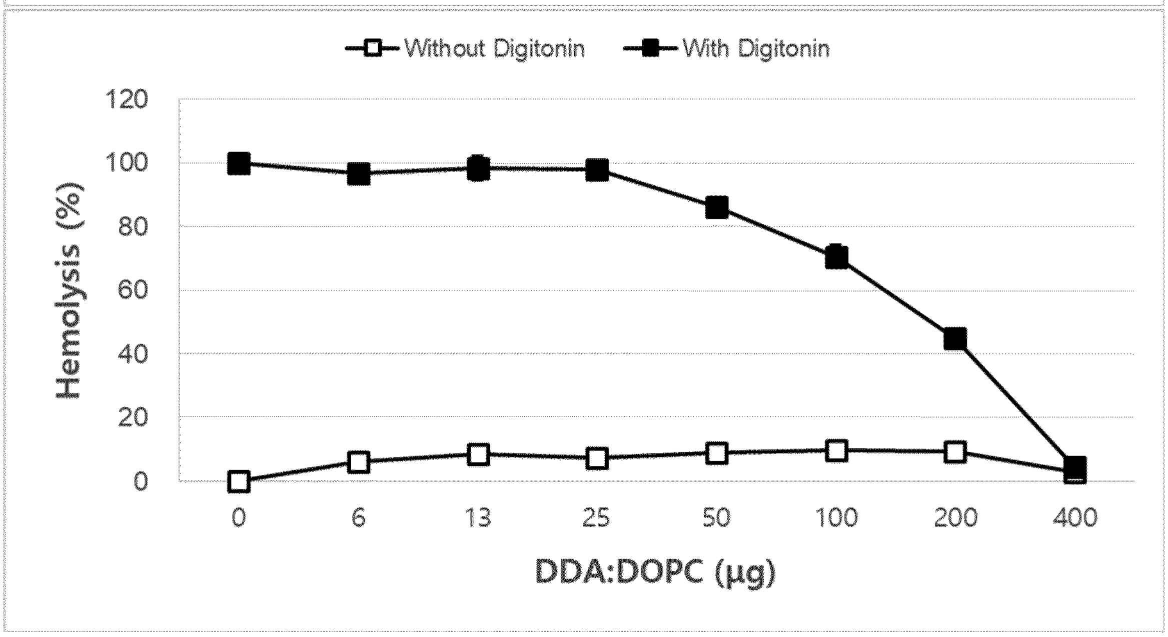
Without Digitonin
With Digitonin
Hemolysis (%)
120
100
80
60
40
20
0
0
6
13
25
50
100
200
400
DDA:DOPC (μg)

FIG. 18

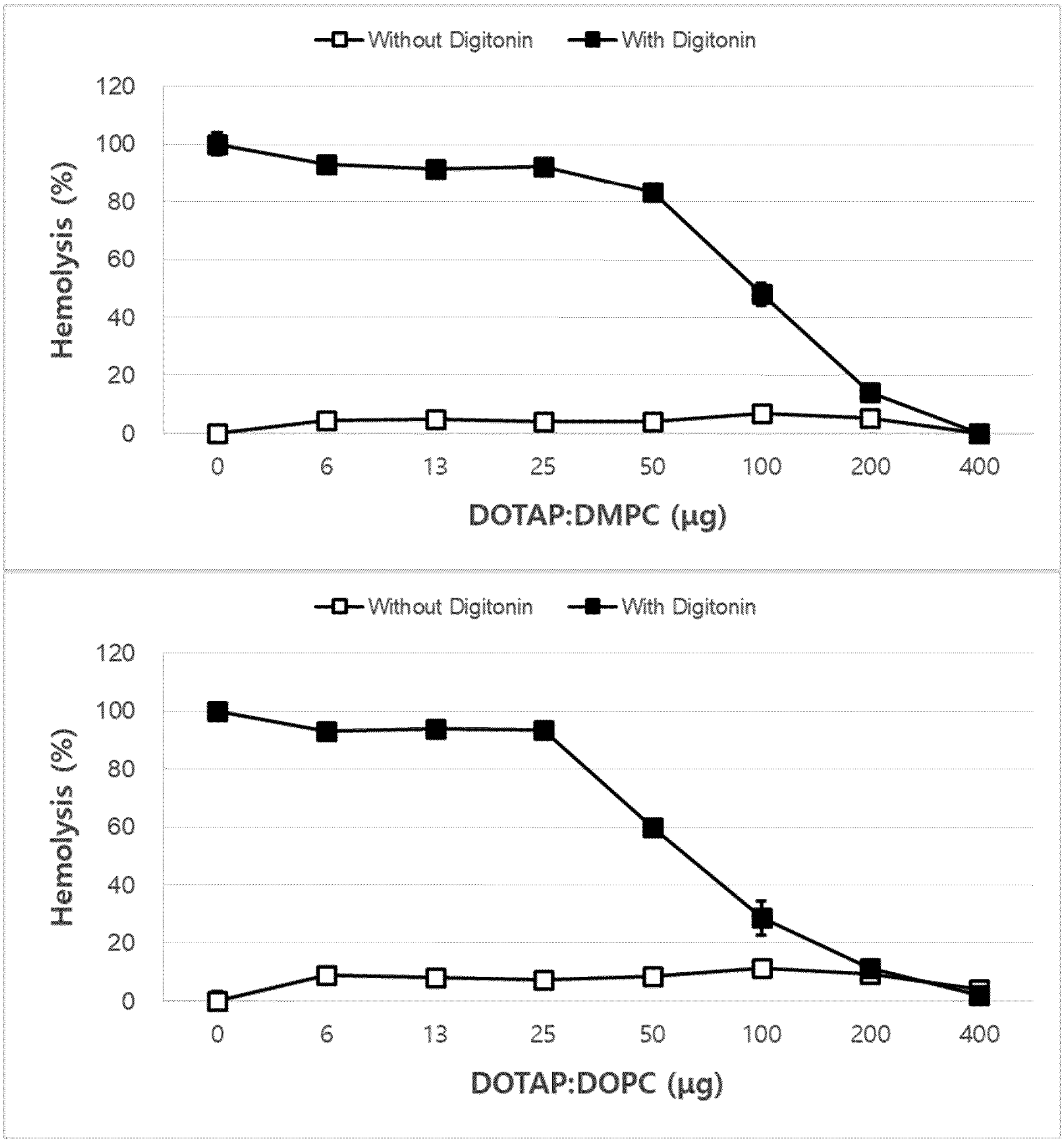
Without Digitonin
With Digitonin
Hemolysis (%)
120
100
80
60
40
20
0
0
6
13
25
50
100
200
400
DOTAP:DMPC (μg)
Without Digitonin
With Digitonin
Hemolysis (%)
120
100
80
60
40
20
0
0
6
13
25
50
100
200
400
DOTAP:DOPC (μg)

FIG. 19

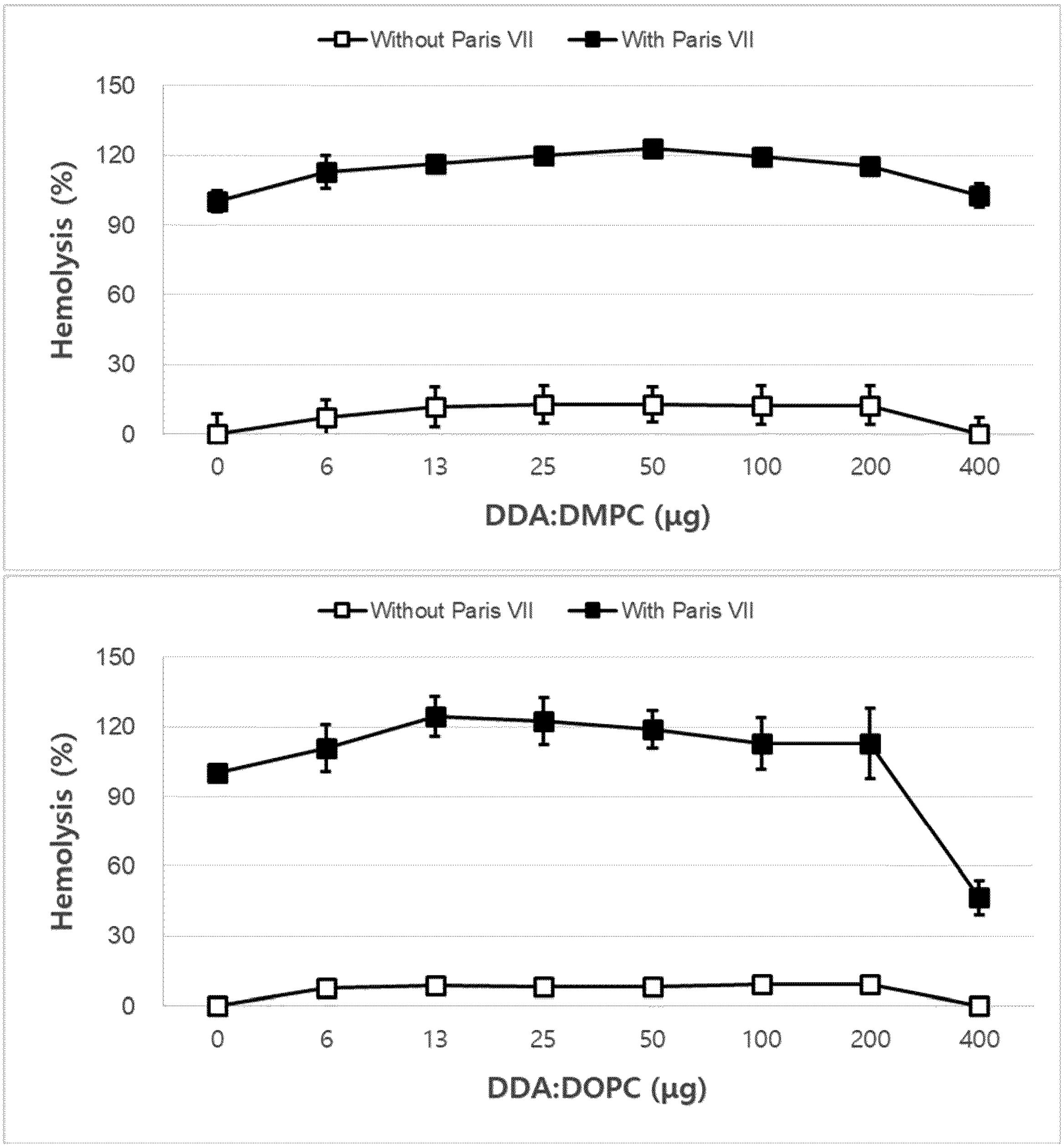
Without Paris VII
With Paris VII
Hemolysis (%)
150
120
90
60
30
0
0
6
13
25
50
100
200
400
DDA:DMPC (μg)
Without Paris VII
With Paris VII
Hemolysis (%)
150
120
90
60
30
0
0
6
13
25
50
100
200
400
DDA:DOPC (μg)

FIG. 20

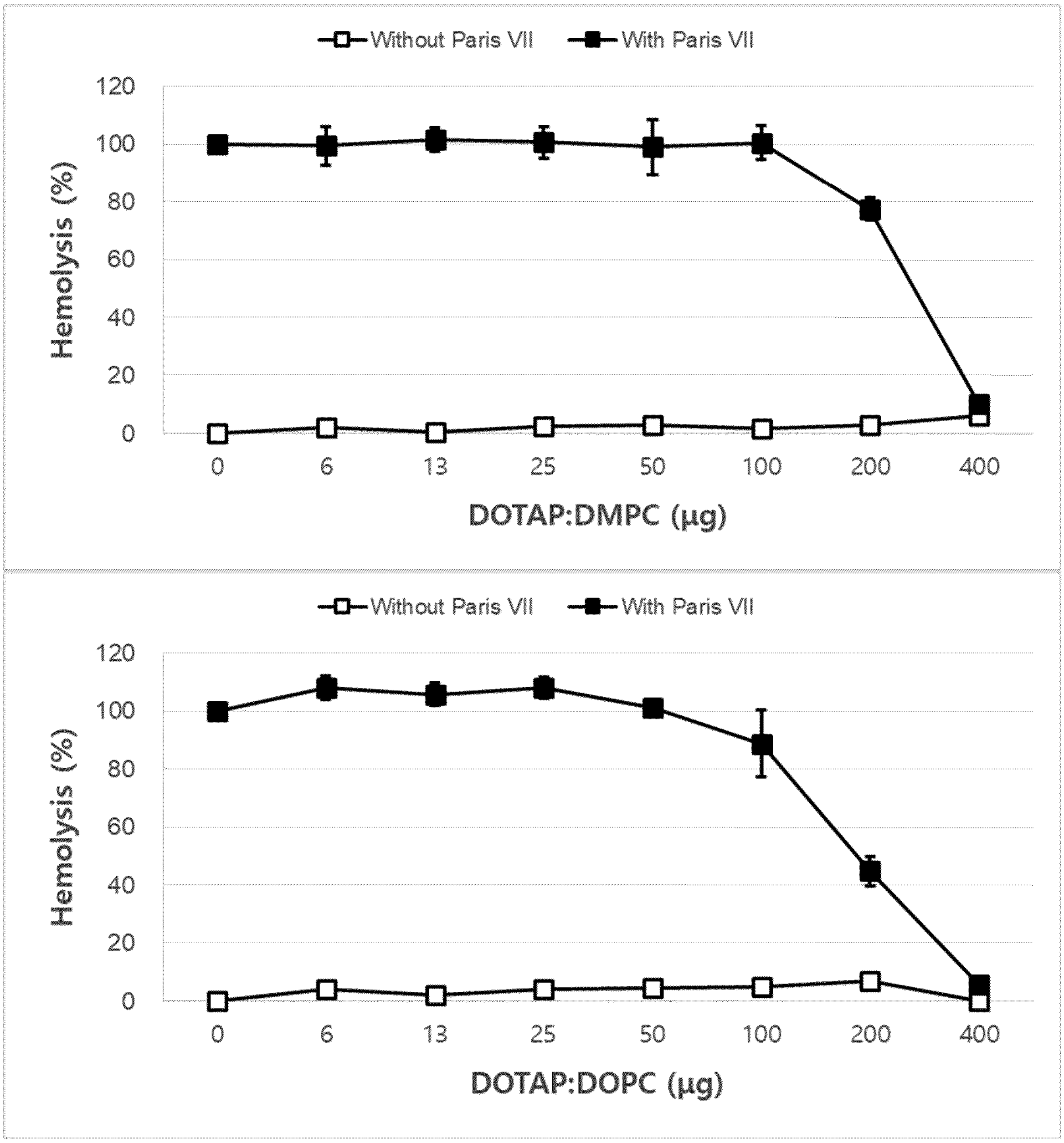
Without Paris VII
With Paris VII
120
100
80
60
40
20
0
Hemolysis (%)
0
6
13
25
50
100
200
400
DOTAP:DMPC (μg)
Without Paris VII
With Paris VII
120
100
80
60
40
20
0
Hemolysis (%)
0
6
13
25
50
100
200
400
DOTAP:DOPC (μg)

FIG. 21

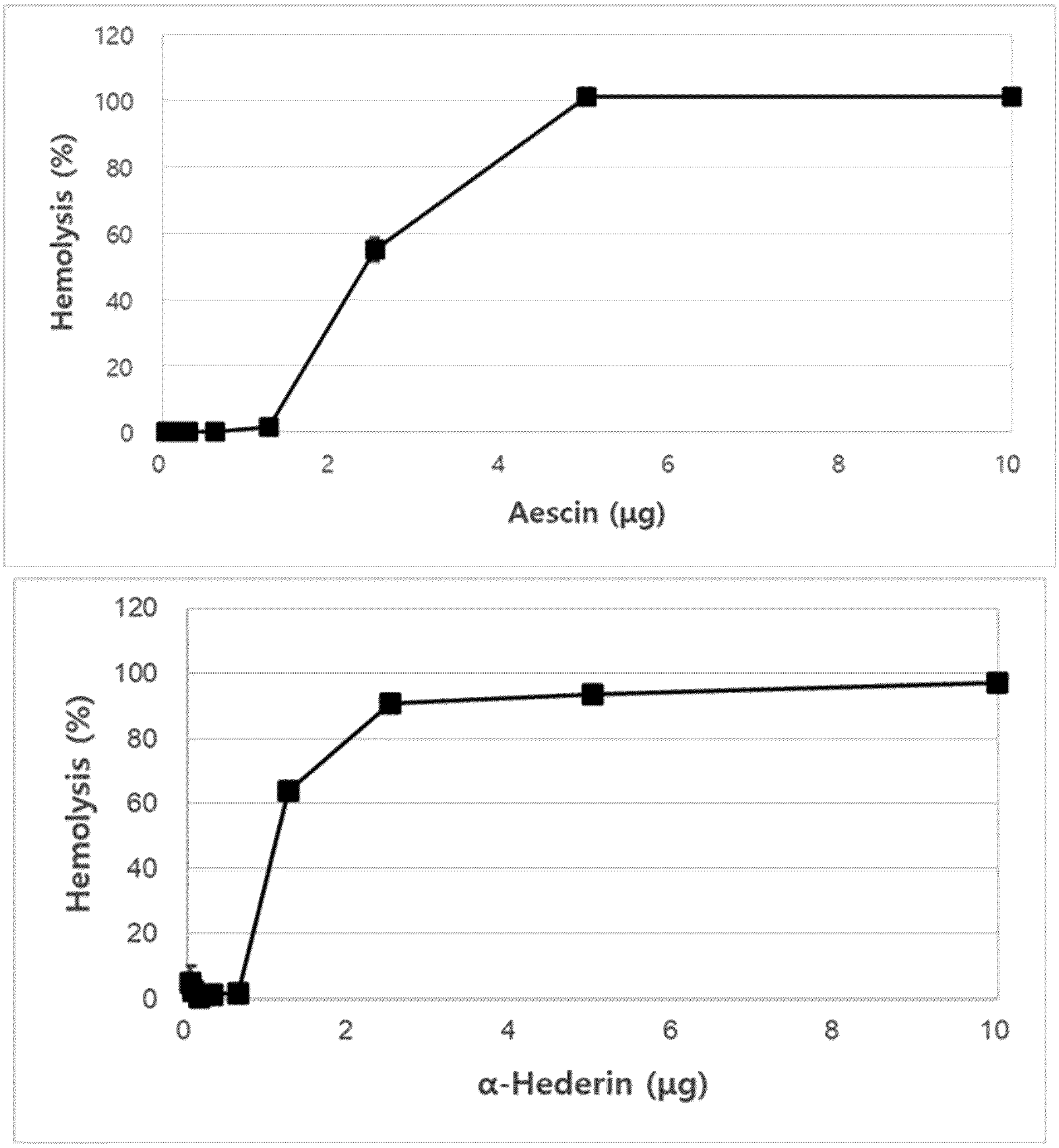
Hemolysis (%)
120
100
80
60
40
20
0
0
2
4
6
8
10
Aescin (μg)
Hemolysis (%)
120
100
80
60
40
20
0
0
2
4
6
8
10
α-Hederin (μg)

FIG. 22

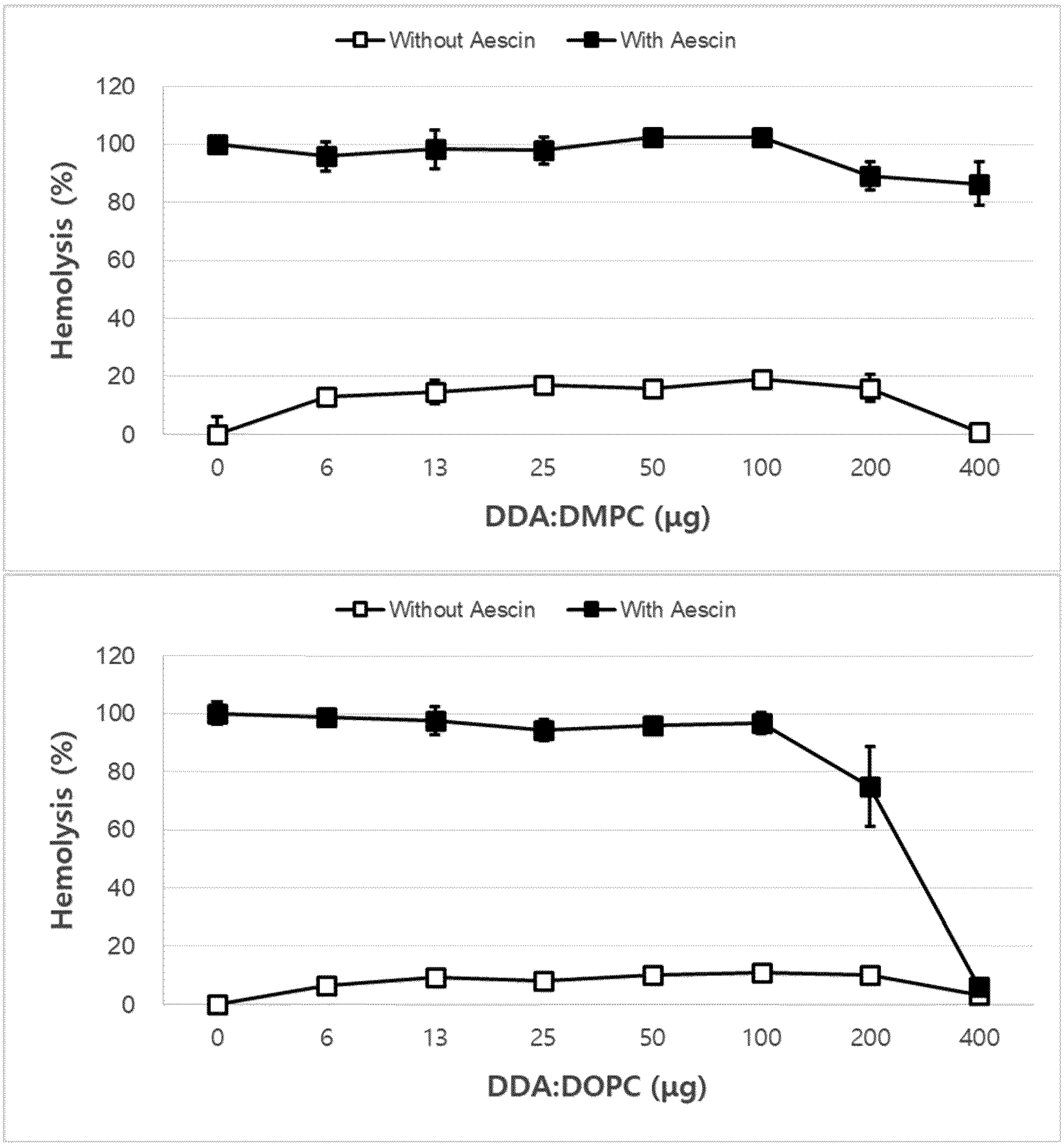
Without Aescin
With Aescin
Hemolysis (%)
120
100
80
60
40
20
0
0
6
13
25
50
100
200
400
DDA:DMPC (μg)
Without Aescin
With Aescin
Hemolysis (%)
120
100
80
60
40
20
0
0
6
13
25
50
100
200
400
DDA:DOPC (μg)

FIG. 23

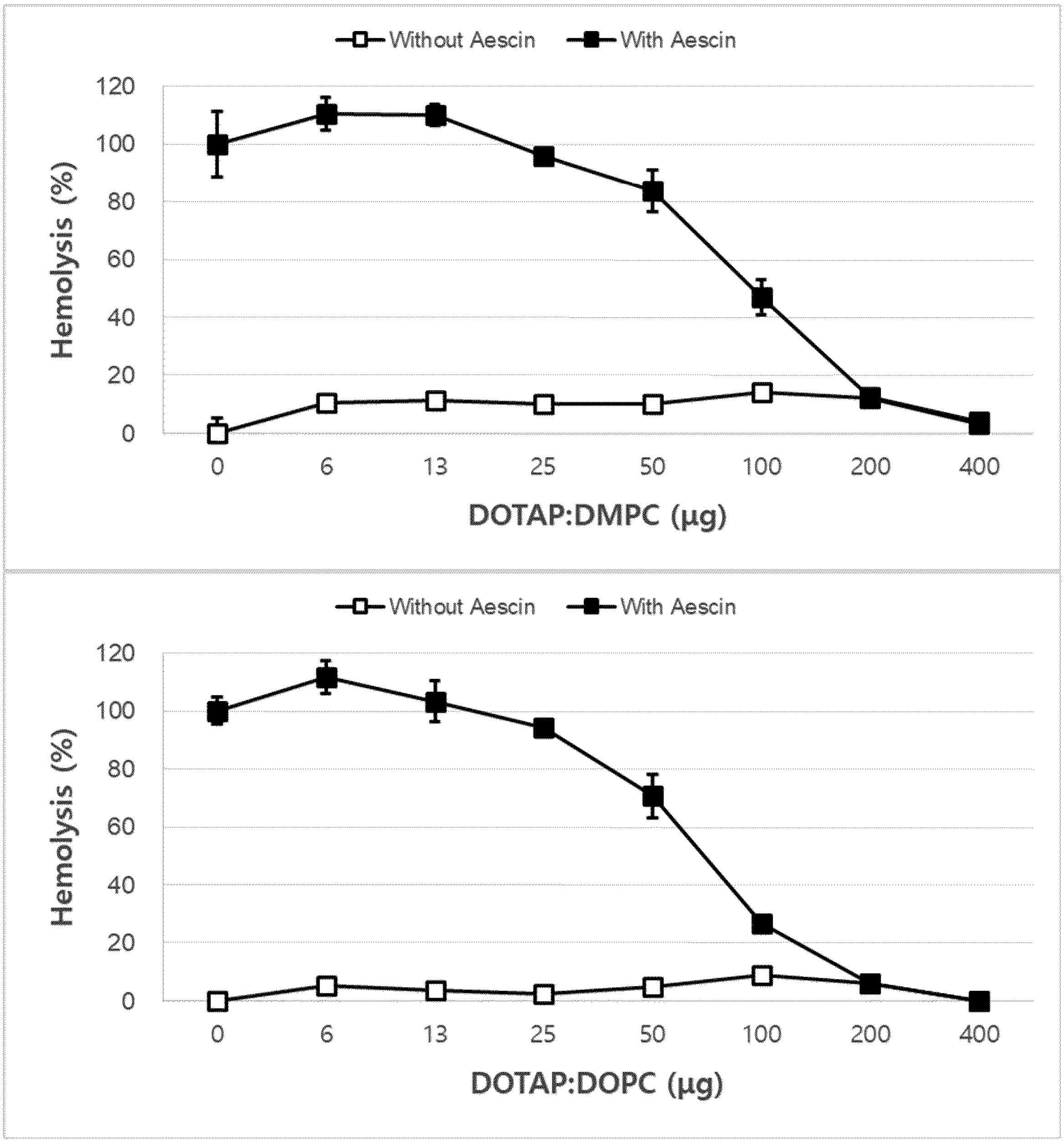
Without Aescin
With Aescin
Hemolysis (%)
120
100
80
60
40
20
0
0
6
13
25
50
100
200
400
DOTAP:DMPC (μg)
Without Aescin
With Aescin
Hemolysis (%)
120
100
80
60
40
20
0
0
6
13
25
50
100
200
400
DOTAP:DOPC (μg)

FIG. 24

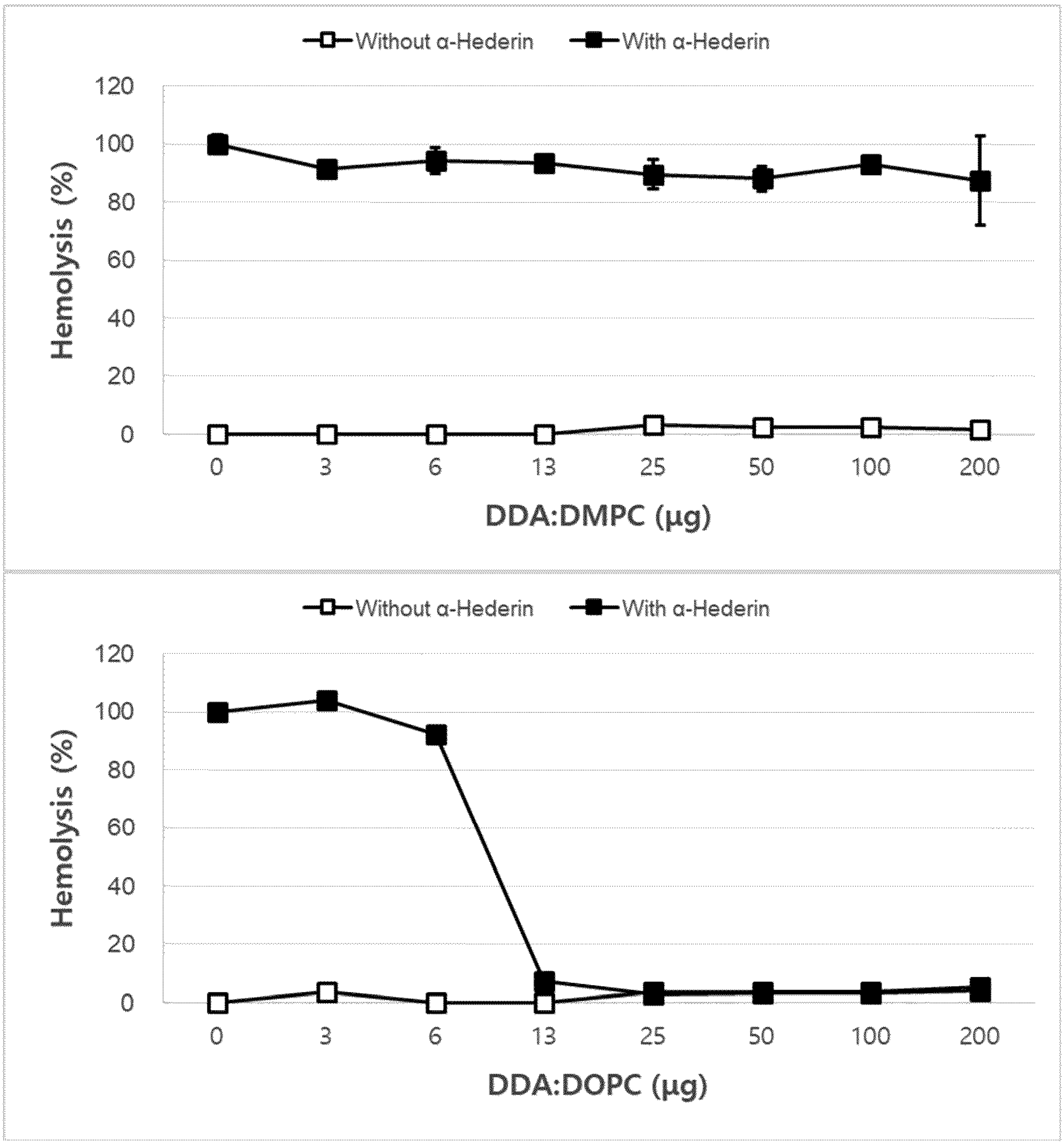

Without α-Hederin
With α-Hederin
Hemolysis (%)
120
100
80
60
40
20
0
0
3
6
13
25
50
100
200
DDA:DMPC (μg)
Without α-Hederin
With α-Hederin
Hemolysis (%)
120
100
80
60
40
20
0
0
3
6
13
25
50
100
200
DDA:DOPC (μg)

FIG. 25

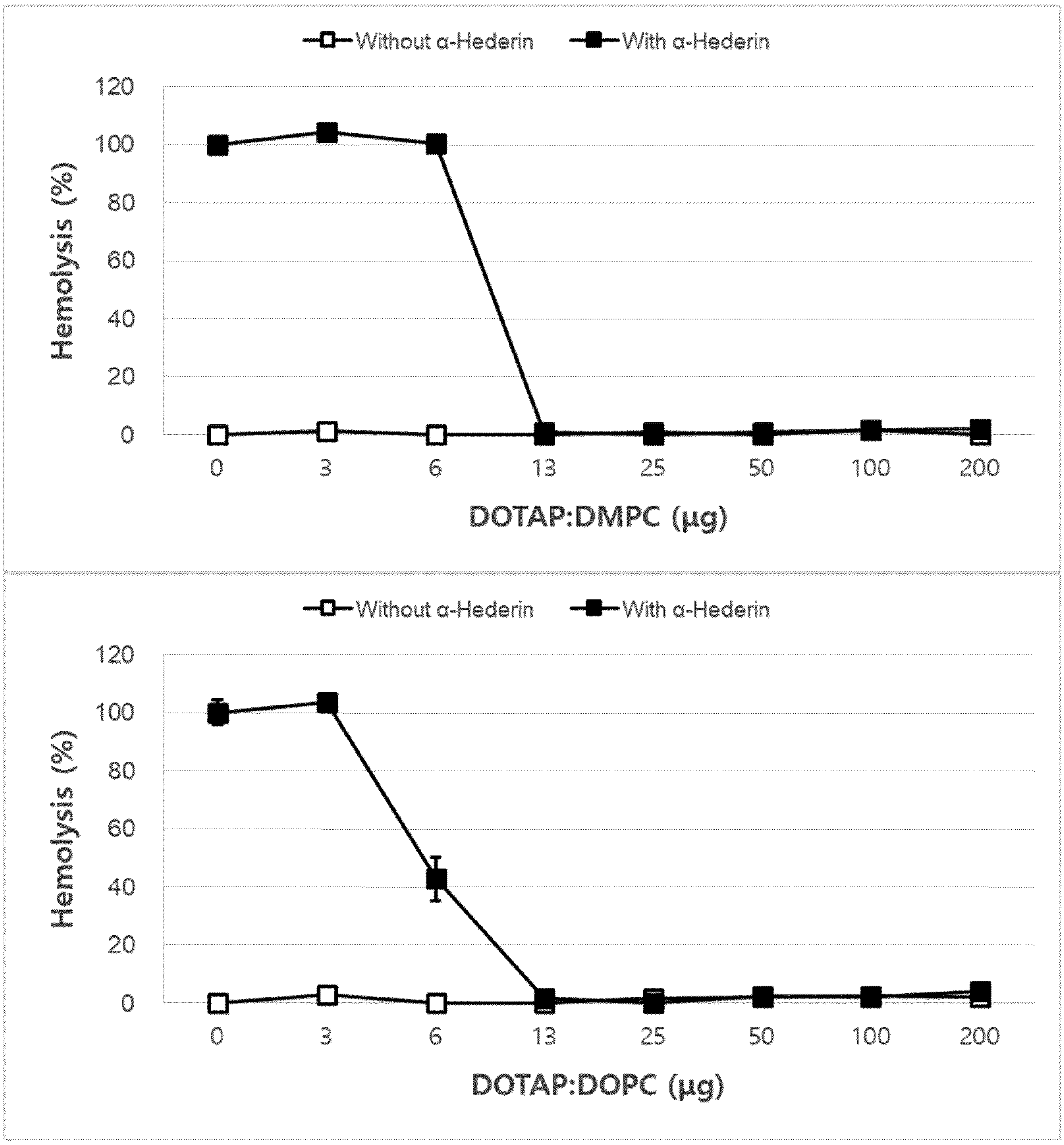
Without α-Hederin
With α-Hederin
120
100
80
60
40
20
0
Hemolysis (%)
0
3
6
13
25
50
100
200
DOTAP:DMPC (μg)
Without α-Hederin
With α-Hederin
120
100
80
60
40
20
0
Hemolysis (%)
0
3
6
13
25
50
100
200
DOTAP:DOPC (μg)

FIG. 26

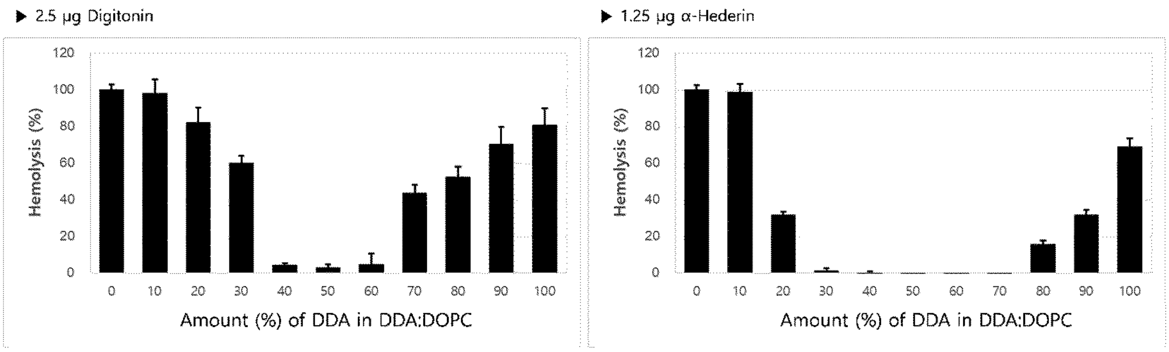


FIG. 27

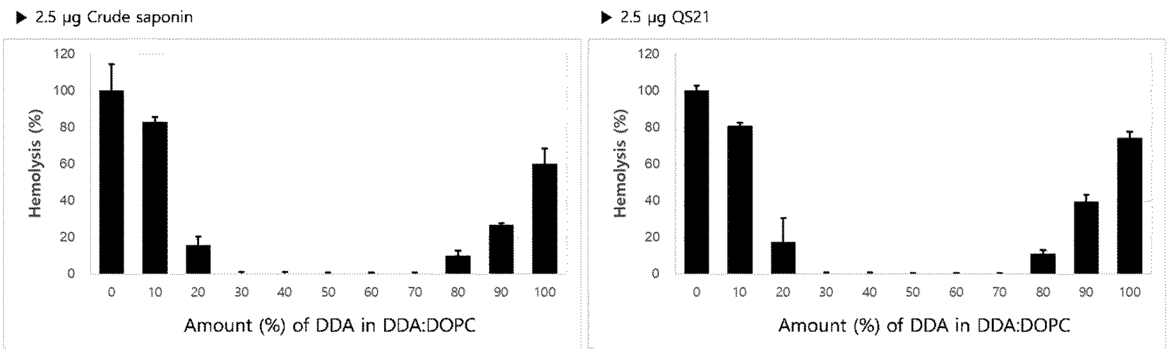


FIG. 28

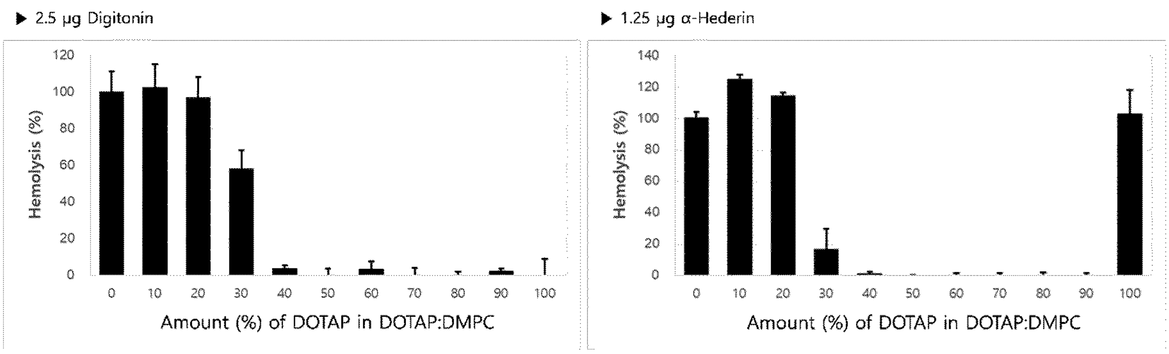

FIG. 29

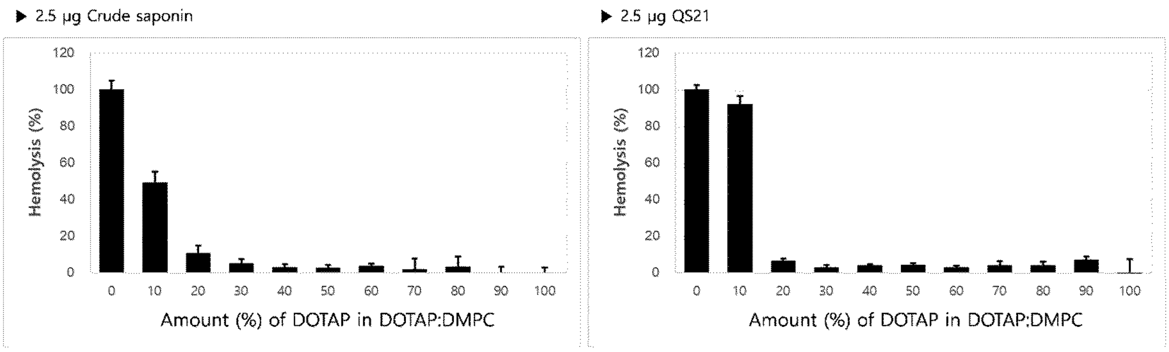


FIG. 30

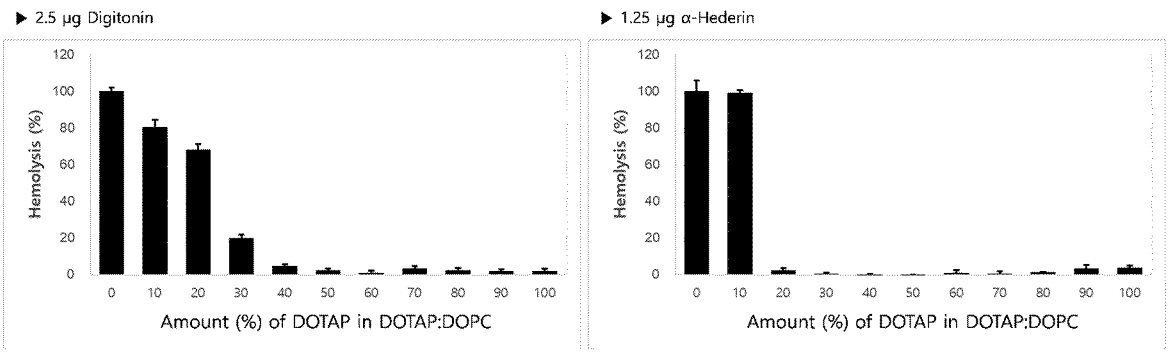


FIG. 31

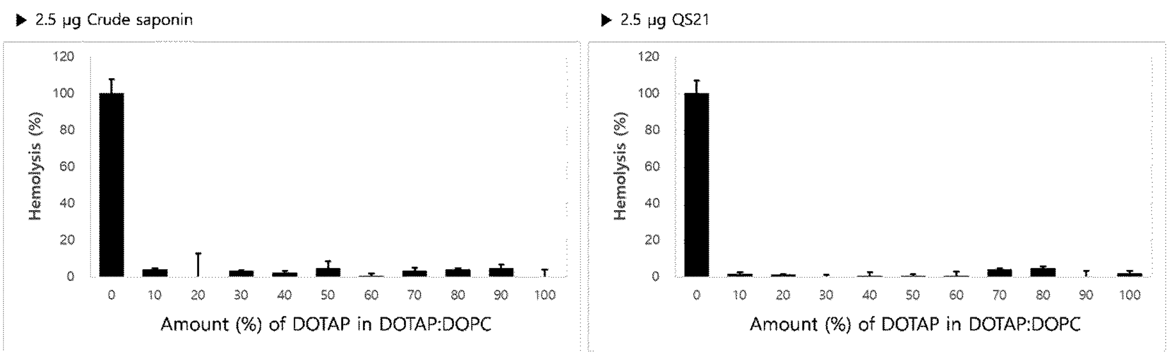

**INTERNATIONAL SEARCH REPORT**

International application No.
**PCT/IB2021/054673**

**A. CLASSIFICATION OF SUBJECT MATTER**

**A61K 9/127**(2006.01)i; **A61K 31/704**(2006.01)i; **A61K 36/185**(2006.01)i; **A61K 47/69**(2017.01)i; **A61P 37/00**(2006.01)i

According to International Patent Classification (IPC) or to both national classification and IPC

**B. FIELDS SEARCHED**

Minimum documentation searched (classification system followed by classification symbols)

A61K 9/127(2006.01); A61K 38/00(2006.01); A61K 39/00(2006.01); A61K 39/12(2006.01); A61K 39/39(2006.01); C07K 16/18(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 사포닌(saponin), 리포좀(liposome), 지질(lipid), 용혈(hemolysis), 약물 전달체 (drug delivery matrix)

**C. DOCUMENTS CONSIDERED TO BE RELEVANT**

| Category* | Citation of document, with indication, where appropriate, of the relevant passages | Relevant to claim No. |
|---|---|---|
| X<br>A | KR 10-2019-0021938 A (EYEGENE INC.) 06 March 2019 (2019-03-06)<br>See claims 1, 3-5 and 11; and paragraphs [0002] and [0146]. | 11-14<br>1-10 |
| X | JP 2020-502258 A (EYEGENE INC.) 23 January 2020 (2020-01-23)<br>See claims 1, 3 and 4; and paragraph [0002]. | 11-14 |
| A | KR 10-2017-0007242 A (THE GOVERNMENT OF THE UNITED STATES OF AMERICA AS REPRESENTED BY THE SECRETARY OF THE ARMY) 18 January 2017 (2017-01-18)<br>See claims 1-3 and 18. | 1-14 |
| A | KR 10-2007-0114854 A (GLAXOSMITHKLINE BIOLOGICALS S.A.) 04 December 2007 (2007-12-04)<br>See claims 1-20. | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2021** | **07 September 2021** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/IB2021/054673**

**C. DOCUMENTS CONSIDERED TO BE RELEVANT**

| Category* | Citation of document, with indication, where appropriate, of the relevant passages | Relevant to claim No. |
|---|---|---|
| A | JP 2014-503475 A (AC IMMUNE S.A.) 13 February 2014 (2014-02-13)<br>See entire document. | 1-14 |

Form PCT/ISA/210 (second sheet) (July 2019)

**INTERNATIONAL SEARCH REPORT**
**Information on patent family members**

International application No.
**PCT/IB2021/054673**

| Patent document cited in search report | | | Publication date (day/month/year) | Patent family member(s) | | | Publication date (day/month/year) |
|---|---|---|---|---|---|---|---|
| KR | 10-2019-0021938 | A | 06 March 2019 | CN | 110461355 | A | 15 November 2019 |
| | | | | CN | 110545840 | A | 06 December 2019 |
| | | | | EA | 201991076 | A1 | 31 October 2019 |
| | | | | EA | 201991081 | A1 | 31 October 2019 |
| | | | | EP | 3533463 | A1 | 04 September 2019 |
| | | | | EP | 3533464 | A1 | 04 September 2019 |
| | | | | JP | 2020-500213 | A | 09 January 2020 |
| | | | | JP | 2020-502258 | A | 23 January 2020 |
| | | | | KR | 10-2042993 | B1 | 11 November 2019 |
| | | | | KR | 10-2086986 | B1 | 10 March 2020 |
| | | | | KR | 10-2086988 | B1 | 10 March 2020 |
| | | | | KR | 20190021939 | A | 06 March 2019 |
| | | | | US | 10874733 | B2 | 29 December 2020 |
| | | | | US | 10918709 | B2 | 16 February 2021 |
| | | | | US | 2019-0255171 | A1 | 22 August 2019 |
| | | | | US | 2019-0290749 | A1 | 26 September 2019 |
| | | | | WO | 2018-080252 | A1 | 03 May 2018 |
| | | | | WO | 2018-080253 | A1 | 03 May 2018 |
| JP | 2020-502258 | A | 23 January 2020 | CN | 110461355 | A | 15 November 2019 |
| | | | | CN | 110545840 | A | 06 December 2019 |
| | | | | EA | 201991076 | A1 | 31 October 2019 |
| | | | | EA | 201991081 | A1 | 31 October 2019 |
| | | | | EP | 3533463 | A1 | 04 September 2019 |
| | | | | EP | 3533464 | A1 | 04 September 2019 |
| | | | | JP | 2020-500213 | A | 09 January 2020 |
| | | | | KR | 10-2042993 | B1 | 11 November 2019 |
| | | | | KR | 10-2086986 | B1 | 10 March 2020 |
| | | | | KR | 10-2086987 | B1 | 10 March 2020 |
| | | | | KR | 10-2086988 | B1 | 10 March 2020 |
| | | | | US | 10874733 | B2 | 29 December 2020 |
| | | | | US | 10918709 | B2 | 16 February 2021 |
| | | | | US | 2019-0255171 | A1 | 22 August 2019 |
| | | | | US | 2019-0290749 | A1 | 26 September 2019 |
| | | | | WO | 2018-080252 | A1 | 03 May 2018 |
| | | | | WO | 2018-080253 | A1 | 03 May 2018 |
| KR | 10-2017-0007242 | A | 18 January 2017 | CA | 2943190 | A1 | 01 October 2015 |
| | | | | CN | 107124869 | A | 01 September 2017 |
| | | | | EP | 3122380 | A1 | 01 February 2017 |
| | | | | JP | 2017-515889 | A | 15 June 2017 |
| | | | | JP | 6608422 | B2 | 20 November 2019 |
| | | | | MX | 2016012168 | A | 25 April 2017 |
| | | | | RU | 2016141622 | A | 25 April 2018 |
| | | | | RU | 2016141622 | A3 | 26 October 2018 |
| | | | | SG | 10201808315 | A | 30 October 2018 |
| | | | | SG | 11201607396 | A | 28 October 2016 |
| | | | | US | 10434167 | B2 | 08 October 2019 |
| | | | | US | 2017-0182152 | A1 | 29 June 2017 |
| | | | | WO | 2015-148648 | A1 | 01 October 2015 |
| KR | 10-2007-0114854 | A | 04 December 2007 | CA | 2370697 | A1 | 26 October 2000 |
| | | | | CA | 2370697 | C | 06 March 2012 |

Form PCT/ISA/210 (patent family annex) (July 2019)

**INTERNATIONAL SEARCH REPORT**
**Information on patent family members**

International application No.
**PCT/IB2021/054673**

| Patent document cited in search report | Publication date (day/month/year) | Patent family member(s) | | | Publication date (day/month/year) |
|---|---|---|---|---|---|
| | | CA | 2425358 | A1 | 25 April 2002 |
| | | CA | 2425358 | C | 21 August 2012 |
| | | CN | 1227030 | C | 16 November 2005 |
| | | CN | 1372473 | A | 02 October 2002 |
| | | CN | 1481255 | A | 10 March 2004 |
| | | CN | 1481255 | C | 10 March 2004 |
| | | CN | 1739800 | A | 01 March 2006 |
| | | CN | 1739800 | C | 01 March 2006 |
| | | CN | 1911443 | A | 14 February 2007 |
| | | CN | 1911443 | C | 14 February 2007 |
| | | CO | 5241279 | A1 | 31 January 2003 |
| | | EP | 1187629 | A2 | 20 March 2002 |
| | | EP | 1187629 | B1 | 22 September 2004 |
| | | EP | 1326638 | A2 | 16 July 2003 |
| | | EP | 1326638 | B1 | 28 November 2007 |
| | | EP | 1326638 | B9 | 20 February 2008 |
| | | EP | 1541170 | A1 | 15 June 2005 |
| | | EP | 1889630 | A1 | 20 February 2008 |
| | | EP | 1889630 | B1 | 23 November 2011 |
| | | EP | 1905449 | A2 | 02 April 2008 |
| | | EP | 1905449 | A3 | 25 March 2009 |
| | | EP | 2266603 | A1 | 29 December 2010 |
| | | EP | 2266603 | B1 | 12 September 2012 |
| | | EP | 2322210 | A1 | 18 May 2011 |
| | | HK | 1044484 | A1 | 25 October 2002 |
| | | HK | 1044484 | B | 29 July 2005 |
| | | HK | 1057992 | A1 | 30 April 2004 |
| | | HK | 1117729 | A1 | 23 January 2009 |
| | | HK | 1147673 | A1 | 19 August 2011 |
| | | HU | 0200815 | A2 | 28 August 2002 |
| | | HU | 0200815 | A3 | 28 July 2004 |
| | | HU | 0402067 | A2 | 28 January 2005 |
| | | HU | 0402067 | A3 | 28 September 2012 |
| | | HU | 229255 | B1 | 28 October 2013 |
| | | HU | 229642 | B1 | 28 March 2014 |
| | | JP | 2002-542203 | A | 10 December 2002 |
| | | JP | 2002-542203 | U | 10 December 2002 |
| | | JP | 2004-511527 | A | 15 April 2004 |
| | | JP | 2007-191491 | A | 02 August 2007 |
| | | JP | 2008-063342 | A | 21 March 2008 |
| | | JP | 2008-285487 | A | 27 November 2008 |
| | | JP | 2011-241222 | A | 01 December 2011 |
| | | JP | 4805242 | B2 | 02 November 2011 |
| | | JP | 4897547 | B2 | 14 March 2012 |
| | | JP | 5307859 | B2 | 02 October 2013 |
| | | KR | 10-0831139 | B1 | 20 May 2008 |
| | | KR | 10-0860893 | B1 | 29 September 2008 |
| | | KR | 10-2002-0067617 | A | 23 August 2002 |
| | | KR | 10-2002-0067617 | A | 23 August 2002 |
| | | KR | 10-2003-0044015 | A | 02 June 2003 |

Form PCT/ISA/210 (patent family annex) (July 2019)

**INTERNATIONAL SEARCH REPORT**
**Information on patent family members**

International application No.
**PCT/IB2021/054673**

| Patent document cited in search report | Publication date (day/month/year) | Patent family member(s) | Publication date (day/month/year) |
|---|---|---|---|
| | | KR 10-2007-0114230 A | 29 November 2007 |
| | | MX PA03003408 A | 30 June 2005 |
| | | MY 127452 A | 29 December 2006 |
| | | NO 331688 B1 | 27 February 2012 |
| | | US 2003-0161834 A1 | 28 August 2003 |
| | | US 2005-0019340 A1 | 27 January 2005 |
| | | US 2008-0095788 A1 | 24 April 2008 |
| | | US 2008-0311156 A1 | 18 December 2008 |
| | | US 6544518 B1 | 08 April 2003 |
| | | US 6558670 B1 | 06 May 2003 |
| | | US 7399472 B2 | 15 July 2008 |
| JP 2014-503475 A | 13 February 2014 | AU 2011-322553 A1 | 18 April 2013 |
| | | AU 2011-322553 A1 | 03 May 2012 |
| | | AU 2011-322553 B2 | 02 July 2015 |
| | | CA 2813833 A1 | 03 May 2012 |
| | | CA 2813833 C | 22 September 2020 |
| | | CN 103189050 A | 03 July 2013 |
| | | CN 103189050 B | 29 September 2017 |
| | | EP 2632434 A1 | 04 September 2013 |
| | | JP 6027011 B2 | 16 November 2016 |
| | | KR 10-1854943 B1 | 04 May 2018 |
| | | KR 10-2014-0009206 A | 22 January 2014 |
| | | TW 201223561 A | 16 June 2012 |
| | | US 2013-0224287 A1 | 29 August 2013 |
| | | US 9687447 B2 | 27 June 2017 |
| | | WO 2012-055933 A1 | 03 May 2012 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NEWMAN MJ et al.** *J. Immunol.,* 1992, vol. 148, 2357-2362
- **SUN HX et al.** *Vaccine,* 2009, vol. 27, 1787-1796
- **KWANG JAE CHO.** *Korean Journal of Otorhinolaryngology-Head and Neck Surgery,* 2007, vol. 50 (7), 562-572
- Remington's Pharmaceutical Sciences. 1995
- Remington's Pharmaceutical Science. Mack Publishing Co,